(19) Europäisches Patentamt
European Patent Office
Office européen des brevets

(11) EP 3 866 802 B1

(12) **EUROPEAN PATENT SPECIFICATION**

(45) Date of publication and mention of the grant of the patent:
**21.08.2024 Bulletin 2024/34**

(21) Application number: **19798788.6**

(22) Date of filing: **16.10.2019**

(51) International Patent Classification (IPC):
***A61K 31/4706*** *(2006.01)* ***A61K 31/4709*** *(2006.01)*
***A61P 25/00*** *(2006.01)* ***A61P 25/28*** *(2006.01)*

(52) Cooperative Patent Classification (CPC):
**A61K 31/4706; A61K 31/4709; A61P 25/00; A61P 25/28;** Y02A 50/30

(86) International application number:
**PCT/US2019/056530**

(87) International publication number:
**WO 2020/081678 (23.04.2020 Gazette 2020/17)**

(54) **QUINONE REDUCTASE 2 INHIBITORS FOR USE AS NEUROPROTECTIVE AGENTS**

CHINON-REDUKTASE-2-INHIBITOREN ZUR VERWENDUNG ALS NEUROPROTEKTIVA

INHIBITEURS DE LA QUINONE RÉDUCTASE 2 DESTINÉS À ÊTRE UTILISÉS EN TANT QU'AGENTS NEUROPROTECTEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2018 US 201862746868 P**

(43) Date of publication of application:
**25.08.2021 Bulletin 2021/34**

(73) Proprietor: **Duke University**
**Durham, North Carolina 27705 (US)**

(72) Inventors:
- **LASCOLA, Christopher D.**
  **Durham, North Carolina 27707 (US)**
- **FITZPATRICK, Jesse Keiser**
  **San Francisco, California 94131 (US)**

(74) Representative: **Marks & Clerk LLP**
**15 Fetter Lane**
**London EC4A 1BW (GB)**

(56) References cited:
**WO-A1-2008/014602 WO-A1-2008/074068**
**WO-A2-2014/066506 US-A1- 2006 074 105**
**US-A1- 2011 190 342 US-A1- 2012 040 993**
**US-A1- 2012 172 376 US-A1- 2015 335 635**
**US-A1- 2017 226 095**

- **STOJANOVICH L ET AL: "Psychiatric manifestations in systemic lupus erythematosus", AUTOIMMUNITY REVIEWS, ELSEVIER, AMSTERDAM, NL, vol. 6, no. 6, 1 June 2007 (2007-06-01), pages 421 - 426, XP025320456, ISSN: 1568-9972, [retrieved on 20070528], DOI: 10.1016/J.AUTREV.2007.02.007**
- **HELENA C CHUI ET AL: "Clinical Criteria for the Diagnosis of Vascular Dementia: A Multicenter Study of Comparability and Interrater Reliability", ARCHIVES OF NEUROLOGY, 1 February 2000 (2000-02-01), United States, pages 191 - 196, XP055653020, Retrieved from the Internet <URL:doi:10.1001/archneur.57.2.191> DOI: 10.1001/archneur.57.2.191**

EP 3 866 802 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

BACKGROUND

Aminoquinolines, with chloroquine (CQ) and hydroxychloroquine (HQ) as prototypes, are quinone reductase 2 (QR2) inhibitors that were originally developed to treat malaria but were subsequently found to have therapeutic efficacy for other indications, including, inter alia, autoimmune diseases such as systemic lupus erythematosis (SLE) and rheumatoid arthritis (RA). Singer et al., "Update on immunosuppressive therapy," Curr. Opin. Rheumatol. 1998, 10:169-173; Wallace, "The use of chloroquine and hydroxychloroquine for non-infectious conditions other than rheumatoid arthritis or lupus: a crucial review," Lupus 1996, 5 Suppl 1: S59-64. In SLE and RA, aminoquinolines are a mainstay of first-line therapy and are often used in combination with other medications. Aminoquinolines not only improve the signs and symptoms of SLE and RA but also have beneficial effects on lipid metabolism and reduce the occurrence of thrombosis. In patients with inflammatory or erosive osteoarthritis, similar benefits are observed. Efficacy has also been shown when used as adjunctive therapy in graft-vs-host disease, cancer, and HIV. Savarino et al., "Effects of chloroquine on viral infections: an old drug against today's diseases?" Lancet Infect. Dis. 2003, 3(11):722-7; Savarino et al., "Risks and benefits of chloroquine use in anticancer strategies," Lancet Oncol. 2006, 7(10):792-3; Sotelo et al., "Adding chloroquine to conventional treatment for glioblastoma multiforme: a randomized, double-blind, placebo-controlled trial," Ann. Intern. Med. 2006, 144(5):337-43.

The potential for chloroquine (CQ) in neuroprotection has been studied previously in preclinical models of stroke, excitotoxic and traumatic injuries, although the therapeutic mechanisms have remained elusive. CQ dramatically limits microglial and PMN migration into injury sites in the brain, decreases reactive astrogliosis and neovascularization, and reduces stroke volumes by 60% in a permanent MCA occlusion model. Giulian et al., "The role of mononuclear phagocytes in wound healing after traumatic injury to adult mammalian brain," J. Neurosci. 1989, 9:4416-4429; Ivanova et al., "Cerebral ischemia enhances polyamine oxidation: identification of enzymatically formed 3-aminopropanal as an endogenous mediator of neuronal and glial cell death," J. Exp. Med. 1998, 188:327-340. CQ also decreases cytokine production by microglial cells in vitro in response to various irritants. Giulian, "Microglia and the immune pathology of Alzheimer disease," Am. J. Hum. Genet. 1999, 65:13-18.

Because some malaria is resistant to CQ, derivative compounds have also been explored. For example, US 2006/0074105 to Ware et al. describes certain quinoline and quinazoline derivatives said to be useful in the treatment of malaria and autoimmune diseases.

Though CQ and HQ are often used clinically as a first-line therapy in autoimmune disorders, their efficacy is limited by serious side effects. The most important and best-characterized toxicity is retinal, where long-term use may lead to "bull's eye maculopathy" and blindness unless dosing is limited. Cardiac toxicity, although rare, may also occur, manifesting either as conduction disturbances (e.g., bundle-branch block) and/or cardiomyopathy in association with congestive heart failure. Electron microscopy of cardiac and retinal biopsies after long-term CQ or HQ therapy reveals pathognomonic cytoplasmic inclusion bodies, understood to be a direct consequence of high drug accumulation in lysosomes (and melanosomes in retina and skin). Remarkably, CQ is capable of accumulating to mM concentration in skin, retinal, renal, and liver cells during therapeutic dosing while plasma concentrations remain < 1 $\mu$M.

In WO 2008/074068 A1, specific heterocyclic compounds are described, as well as processes for their preparation and their use as pharmaceutical or veterinary agents in particular for the treatment, amelioration and/or prophylaxis of conditions caused by or associated with unbalanced metal levels and/or oxidative stress, such as neurological conditions and cellular proliferative disorders, for example Alzheimer's disease, Parkinson's disease, Huntington's disease or brain cancer or tumours.

In US 2006/074105 A1, specific compositions and methods of inhibiting quinone reductase 2 (QR2) are described. The methods are described as useful in the treatment of malaria and autoimmune diseases. The compositions comprise quinoline and quinazoline derivatives. Methods for inhibiting the activity of QR2 by contacting the enzyme with one or more of the compositions are also described.

In US 2017/226095 A1, the manufacture and use of specific compounds or pharmaceutically acceptable salts thereof for preventing, inhibiting or treating cancer, AIDS and/or premature aging is described.

In US 2012/040993 A1, multifunctional compounds are described, which comprise two or more functional moieties selected from: (i) a moiety that imparts an iron chelator function; (ii) a moiety that imparts a neuroprotective function; (iii) a moiety that imparts combined antiapoptotic, neuroprotective and/or neurorestorative functions; (iv) a moiety that imparts brain monoamine oxidase (MAO) inhibition, preferably with little or no MAO inhibition in liver and small intestine; (v) a moiety that imparts cholinesterase inhibitory function; and (vi) a moiety that imparts an N-methyl-D-aspartic acid receptor (NMDAR) inhibition, and pharmaceutically acceptable salts and optical isomers thereof. The multifunctional compounds are described as useful in the treatment or prevention of diseases, disorders or conditions that can be prevented and/or treated by iron chelation therapy, and/or neuroprotection and/or neurorestoration, and/or apoptosis inhibition and/or MAO inhibition and/or cholinesterase inhibition and/or NMADR inhibition.

In US 2015/335635 A1, a method is described for the treatment of a neurological condition in a subject which comprises administering to a subject in need thereof a therapeutically effect amount of a specific compound or pharmaceutically acceptable salts, hydrates, or solvates thereof.

In WO 2008/014602 A1, specific quinoline derivatives are described as active CLK-1 inhibitors. Pharmaceutical compositions comprising such derivatives and methods for the prophylaxis and/or treatment of disorders or their associated symptoms for which the inhibition of CLK-1 is beneficial are also described.

In US 2011/190342 A1, specific quinoline derivatives as well as their pharmaceutically acceptable salts are described, as well as a process for the preparation of such compounds. The compounds are described as being glycine B antagonists and therefore useful for the control and prevention of various disorders, including neurological disorders.

In US 2012/172376 A1, specific heterocyclic compounds are described as useful for inhibiting glycogen synthase kinase 3 (GSK-3). Compositions containing the compounds, their use for preparing a medicament for the treatment of a medical disorder susceptible to the treatment with a compound that modulates, preferably inhibits, the activity of glycogen synthase kinase 3β, and methods of treatment of medical disorders susceptible to treatment with a compound that modulates glycogen synthase kinase 3β activity using the compounds are also described.

In WO 2014/066506 A2, specific compounds, pharmaceutical compositions, and methods of using such compounds to treat or prevent diseases or disorders associated with or mediated by JAMM proteins are described.

There remains a need to develop additional aminoquinoline quinone reductase 2 (QR2) inhibitors, particularly that also have diminished lysosomal accumulation in order to reduce toxicity.

## SUMMARY

The present invention is defined in the appended claims.

Provided herein according to some embodiments is a compound of **Formula I:**

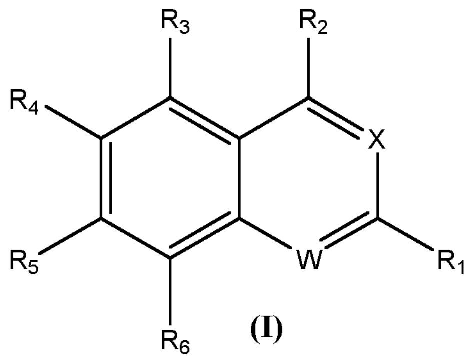

**(I)**

wherein:

- WisN;
- X is $CR_{14}$;
- $R_1$ is H or trifluoromethyl;
- $R_2$ is $NR_7R_8$, $OR_{11}$, $SR_{12}$, or alkyl;
- $R_3$ is H or $OR_{13}$;
- $R_4$ is H or methoxy;
- $R_5$ is H, Cl, or trifluoromethyl;
- $R_6$ is H or trifluoromethyl;
- $R_7$ is H, $C_{1-5}$ alkyl, heteroarylalkyl, cycloalkyl, heterocycloalkyl, heterocyclo, aryl, heteroaryl, ureido, thioureido, alkenyl, alkynyl, amido, amino, alkoxy, alkylamino, alkylphosphonate, alkylnitrile, alkylhalo, or alkylhalo optionally substituted with $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkylhalo;
- $R_8$ is H, $C_{1-5}$ alkyl, cycloalkyl, heterocycloalkyl, aryl, heteroaryl, ureido, thioureido, alkenyl, alkynyl, amido, amino, alkoxy, alkylamino, alkylphosphonate, alkylnitrile, alkylhalo or alkylhalo optionally substituted with $C_{1-5}$ alkyl, cycloalkyl, cycloalkenyl, heterocycloalkyl, heteroaryl, alkenyl, alkynyl, amido, alkoxy, alkylhydroxy, halo, hydroxyl, carboxylate, alkylcarboxylate, acylazido, sulfonamide or alkylhalo;
- $R_9$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, aryl, heteroaryl or alkylamino;
- $R_{10}$ is H, O, $C_{1-5}$ alkyl, cycloalkyl, heterocycloalkyl, aryl, heteroaryl, alkylamino, alkylnitrile or alkylphosphonate

optionally substituted with $C_{1-5}$ alkyl, cycloalkyl, heterocycloalkyl, heteroaryl, or alkylamino;
$R_{11}$ is alkyl, aryl or heteroaryl optionally substituted with alkyl, haloalkyl, aryl or heteroaryl;
$R_{12}$ is alkyl, aryl or heteroaryl optionally substituted with alkyl, haloalkyl, aryl or heteroaryl;
$R_{13}$ is alkyl or aryl optionally substituted with alkyl or haloalkyl; and
$R_{14}$ is H or aryl;
or a pharmaceutically acceptable salt thereof,
for use in treating acute neural injury.

In some embodiments, $R_1$ is H. In some embodiments, $R_2$ is $NR_7R_8$. In some embodiments, $R_3$ is H. In some embodiments, $R_4$ is H. In some embodiments, $R_5$ is Cl. In some embodiments, $R_6$ is H. In some embodiments, $R_7$ is H. In some embodiments, $R_8$ is $C_{1-5}$ alkyl substituted with heteroaryl. In some embodiments, $R_{14}$ is H.

In some embodiments, the compound has a positive log D value at approximately pH 4 to pH 5.

In some embodiments, the compound is a compound of **Formula I(a)**:

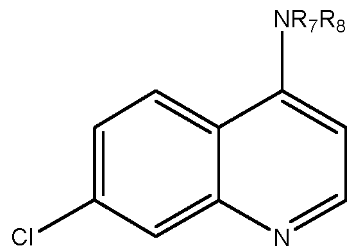

I(a)

wherein $R_7$ and $R_8$ are each independently H or $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, e.g., $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkylhalo).

In some embodiments, one of $R_7$ and $R_8$ is hydrogen, and the other is $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, e.g., $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo).

In some embodiments, the compound has a positive log D value at approximately pH 4 to pH 5.

Also provided is a compound of **Formula II:**

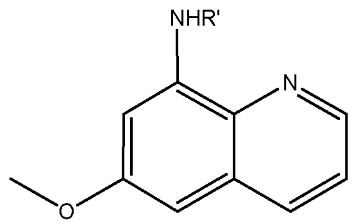

II

wherein R' is selected from the group consisting of pyridin-2-ylmethyl, pyridin-3-ylmethyl, 1-benzylpiperidin-4-yl, 4-cyano-2,2-diethylbutyl, 2-chlorocyclopentyl, 4-(diethylamino)butan-2-yl, 1-(furan-2-yl)ethyl, 1-cyclopropylethyl, 1-ethylpiperidin-4-yl, 5-amino-2,2-diethylpentyl, and 2-(diethylphosphoryl)-1 -methylethyl,

or a pharmaceutically acceptable salt thereof,
for use in treating acute neural injury in a subject in need thereof.

In some embodiments, the compound has a positive log D value at approximately pH 4 to pH 5.

In some embodiments, the acute neural injury comprises traumatic brain injury. In some embodiments, the

acute neural injury comprises subarachnoid hemorrhage. In some embodiments, the acute neural injury comprises post-operative cognitive deficit. In some embodiments, the acute neural injury comprises hypoxic brain injury. In some embodiments, the acute neural injury comprises ischemic brain injury.

Also provided is an active compound as taught herein for use in a method of treatment for an acute neural injury. Further provided is the use of an active compound as taught herein for the preparation of a medicament for the treatment of an acute neural injury. In some embodiments, the acute neural injury comprises traumatic brain injury. In some embodiments, the acute neural injury comprises subarachnoid hemorrhage. In some embodiments, the acute neural injury comprises post-operative cognitive deficit. In some embodiments, the acute neural injury comprises hypoxic brain injury. In some embodiments, the acute neural injury comprises ischemic brain injury.

Further provided is a compound of **Formula I:**

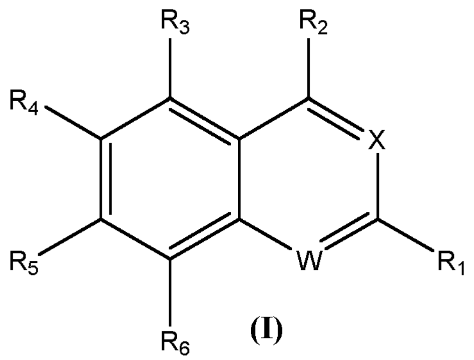

(I)

wherein:

WisN;
X is $CR_{14}$;
$R_1$ is H or trifluoromethyl;
$R_2$ is $NR_7R_8$, $OR_{11}$, $SR_{12}$, or alkyl;
$R_3$ is H or $OR_{13}$;
$R_4$ is H or methoxy;
$R_5$ is H, Cl, or trifluoromethyl;
$R_6$ is H or trifluoromethyl;
$R_7$ is H, $C_{1-5}$ alkyl, heteroarylalkyl, cycloalkyl, heterocycloalkyl, aryl, heterocyclo, heteroaryl, ureido, thioureido, alkenyl, alkynyl, amido, amino, alkoxy, alkylamino, alkylphosphonate, alkylnitrile, alkylhalo, or alkylhalo optionally substituted with $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkylhalo;
$R_8$ is H, $C_{1-5}$ alkyl, cycloalkyl, heterocycloalkyl, aryl, heteroaryl, ureido, thioureido, alkenyl, alkynyl, amido, amino, alkoxy, alkylamino, alkylphosphonate, alkylnitrile, alkylhalo or alkylhalo optionally substituted with $C_{1-5}$ alkyl, cycloalkyl, cycloalkenyl, heterocycloalkyl, heteroaryl, alkenyl, alkynyl, amido, alkoxy, alkylhydroxy, halo, hydroxyl, carboxylate, alkylcarboxylate, acylazido, sulfonamide or alkylhalo;
$R_9$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, aryl, heteroaryl or alkylamino;
$R_{10}$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, heteroaryl, or alkylamino;
$R_{11}$ is alkyl, aryl or heteroaryl optionally substituted with alkyl, haloalkyl, aryl or heteroaryl;
$R_{12}$ is alkyl, aryl or heteroaryl optionally substituted with alkyl, haloalkyl, aryl or heteroaryl;
$R_{13}$ is alkyl or aryl optionally substituted with alkyl or haloalkyl; and
$R_{14}$ is H or aryl;
or a pharmaceutically acceptable salt thereof,
for use in treating vascular dementia.

In some embodiments, $R_1$ is H. In some embodiments, $R_2$ is $NR_7R_8$. In some embodiments, $R_3$ is H. In some embodiments, $R_4$ is H. In some embodiments, $R_5$ is Cl. In some embodiments, $R_6$ is H. In some embodiments, $R_7$ is H. In some embodiments, $R_8$ is $C_{1-5}$ alkyl substituted with heteroaryl. In some embodiments, $R_{14}$ is H.

In some embodiments, the compound has a positive log D value at approximately pH 4 to pH 5.

In some embodiments, the compound is a compound of **Formula I(a):**

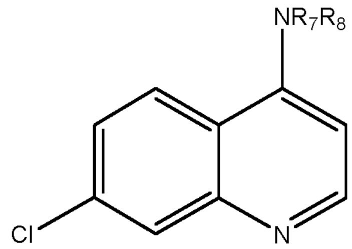

I(a)

wherein $R_7$ and $R_8$ are each independently H or $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, e.g., $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo).

In some embodiments, one of $R_7$ and $R_8$ is hydrogen, and the other is $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, e.*g.*, $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo).

In some embodiments, the compound has a positive log D value at approximately pH 4 to pH 5.

Also provided is a compound of **Formula II:**

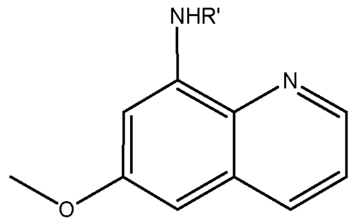

II

wherein R' is selected from the group consisting of pyridin-2-ylmethyl, pyridin-3-ylmethyl, 1-benzylpiperidin-4-yl, 4-cyano-2,2-diethylbutyl, 2-chlorocyclopentyl, 4-(diethylamino)butan-2-yl, 1-(furan-2-yl)ethyl, 1-cyclopropylethyl, 1-ethylpiperidin-4-yl, 5-amino-2,2-diethylpentyl, and 2-(diethylphosphoryl)-1-methylethyl,

or a pharmaceutically acceptable salt thereof,
for use in treating vascular dementia in a subject in need thereof.

In some embodiments, the compound has a positive log D value at approximately pH 4 to pH 5.

Also provided is an active compound as taught herein for use in a method of treatment for vascular dementia. Further provided is the use of an active compound as taught herein for the preparation of a medicament for the treatment of vascular dementia.

Still further provided is compound of **Formula I:**

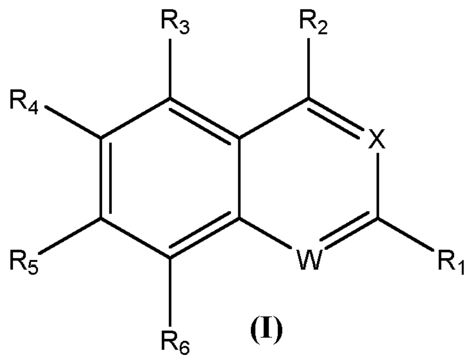

(I)

wherein:

WisN;
X is $CR_{14}$;
$R_1$ is H or trifluoromethyl;
$R_2$ is $NR_7R_8$, $OR_{11}$, $SR_{12}$, or alkyl;
$R_3$ is H or $OR_{13}$;
$R_4$ is H or methoxy;
$R_5$ is H, Cl, or trifluoromethyl;
$R_6$ is H or trifluoromethyl;
$R_7$ is H, $C_{1-5}$ alkyl, heteroarylalkyl, cycloalkyl, heterocycloalkyl, heterocyclo, aryl, heteroaryl, ureido, thioureido, alkenyl, alkynyl, amido, amino, alkoxy, alkylamino, alkylphosphonate, alkylnitrile, alkylhalo, or alkylhalo optionally substituted with $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkylhalo;
$R_8$ is H, $C_{1-5}$ alkyl, cycloalkyl, heterocycloalkyl, aryl, heteroaryl, ureido, thioureido, alkenyl, alkynyl, amido, amino, alkoxy, alkylamino, alkylphosphonate, alkylnitrile, alkylhalo or alkylhalo optionally substituted with $C_{1-5}$ alkyl, cycloalkyl, cycloalkenyl, heterocycloalkyl, heteroaryl, alkenyl, alkynyl, amido, alkoxy, alkylhydroxy, halo, hydroxyl, carboxylate, alkylcarboxylate, acylazido, sulfonamide or alkylhalo;
$R_9$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, aryl, heteroaryl or alkylamino;
$R_{10}$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, heteroaryl, or alkylamino;
$R_{11}$ is alkyl, aryl or heteroaryl optionally substituted with alkyl, haloalkyl, aryl or heteroaryl;
$R_{12}$ is alkyl, aryl or heteroaryl optionally substituted with alkyl, haloalkyl, aryl or heteroaryl;
$R_{13}$ is alkyl or aryl optionally substituted with alkyl or haloalkyl; and
$R_{14}$ is H or aryl;
or a pharmaceutically acceptable salt thereof,
for use in treating central nervous system (CNS) lupus in a subject in need thereof.

In some embodiments, $R_1$ is H. In some embodiments, $R_2$ is $NR_7R_8$. In some embodiments, $R_3$ is H. In some embodiments, $R_4$ is H. In some embodiments, $R_6$ is H. In some embodiments, $R_7$ is H. In some embodiments, $R_8$ is $C_{1-5}$ alkyl substituted with heteroaryl. In some embodiments, $R_{14}$ is H.

In some embodiments, the compound has a positive log D value at approximately pH 4 to pH 5.

In some embodiments, the compound is a compound of **Formula I(a):**

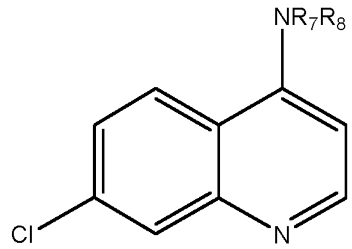

I(a)

wherein $R_7$ and $R_8$ are each independently H or $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, *e.g.*, $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo).

In some embodiments, one of $R_7$ and $R_8$ is hydrogen, and the other is $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, e.g., $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo).

In some embodiments, the compound has a positive log D value at approximately pH 4 to pH 5.

Also provided is a compound of **Formula II:**

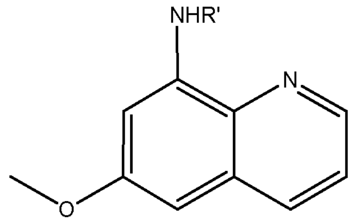

II

wherein R' is selected from the group consisting of pyridin-2-ylmethyl, pyridin-3-ylmethyl, 1-benzylpiperidin-4-yl, 4-cyano-2,2-diethylbutyl, 2-chlorocyclopentyl, 4-(diethylamino)butan-2-yl, 1-(furan-2-yl)ethyl, 1-cyclopropylethyl, 1-ethylpiperidin-4-yl, 5-amino-2,2-diethylpentyl, and 2-(diethylphosphoryl)-1-methylethyl,

or a pharmaceutically acceptable salt thereof,
for use in treating CNS lupus in a subject in need thereof.

In some embodiments, the compound has a positive log D value at approximately pH 4 to pH 5.
Also provided is an active compound as taught herein for use in a method of treatment for CNS lupus. Further provided is the use of an active compound as taught herein for the preparation of a medicament for the treatment of CNS lupus.

BRIEF DESCRIPTION OF THE DRAWINGS

**FIG. 1A-FIG. 1B:** FIG. 1A shows a logD graph of chloroquine, and FIG. 1B shows a logD graph of hydroxychloroquine. The shaded regions in the figures represent the potential range of lysosomal pH encountered *in vivo.* At lysosomal pH, the logD of both chloroquine and hydroxychloroquine are substantially negative, reflecting the accumulated charge of these molecules and their loss of membrane permeability.

**FIG. 2A-FIG. 2E** show the logD graphs of Example Compounds A-E, respectively. Note that for each of the Example Compounds, log D values remain positive at lysosomal pH (between pH 4 and 5).

**FIG. 3A-FIG. 3C:** FIG. 3A provides diffusion weighted magnetic resonance imaging (DW-MRI) images of transient middle cerebral artery occlusion (MCAO) stroke evolution in mice at 4 hr (left) and 24 hr (right). FIG. 3B provides

neurological scores (left) and rotorod assessment (right) with chloroquine (CQ) versus vehicle following MCAO. FIG. 3C shows cortical (left) and subcortical stroke volumes at three days, comparing Example Compound E (7C-4MAQ), chloroquine (CQ), QRII null mice, QRII null mice littermates, and vehicle.

**FIG. 4** presents the results of rotorod (left) and Morris water maze (right) performance following TBI, comparing Example Compound E (7C-4MAQ) to chloroquine (CQ) and vehicle.

**FIG. 5** shows T2 and susceptibility-weighted images (SWI) of hemorrhage in the right basal ganglia in the intracranial hemorrhage model.

**FIG. 6** shows neuronal apoptosis after deep hypothermic circulatory arrest (DHCA). TUNEL analysis in cortex and hippocampus 48 hours after DHCA in rats treated with CQ (25mg/kg, horizontal shaded bar), PBS (equal volume, black bar), QR2 inhibitor 7C-4MAQ (25mg/kg, vertical shaded bar) or vehicle (50% DMSO, open bar).

**FIG. 7** shows neuronal necrosis after DHCA. Acid fuchsin-celestin blue staining in cortex and hippocampus 48 hours after DHCA in rats treated with CQ (25mg/kg, horizontal shaded bar), PBS (equal volume, black bar), QR2 inhibitor 7C-4MAQ (25mg/kg, vertical shaded bar) or vehicle (50% DMSO, open bar).

**FIG. 8** shows neurological outcome analyzed by neuroscore on postoperative days (POD) 1 and 2 in rats treated with QR2 inhibitor 7C-4MAQ (25mg/kg, open bar) or 50% DMSO (solid bar) 2 hours before CPOB/DHCA.

**FIG. 9** shows global brain perfusion as measured by MRI ADC-perfusion. A, ADC-perfusion intensity of sham mice compared to a pooled group of all mice with BCAS at day three and 32. 100% perfusion was defined as average ADC-perfusion intensity of sham group. Perfusion of BCAS mice (n=17) was significantly less than sham mice (n=5) at day three (**$p < 0.01$), but perfusion rebounded to normal levels by day 32. B, representative colorized ADC-perfusion MR sequence superimposed onto a greyscale coronal T2 weighted sequence three days following BCAS surgery. Note the increased perfusion (increased ADC-perfusion signal intensity) in the sham brain compared to other treatment groups. C, ADC-perfusion at day three and 32 by treatment group. Perfusion in the sham group (n = 5) was significantly higher than all other groups on day three ($p < 0.05$, group effect; Sham×N-MCQ, n = 4, $p < 0.01$; Sham×CQ, n = 5, $p < 0.01$; Sham×Vehicle, n = 9, $p < 0.05$).Values represent averages ±SEM. *$p < 0.05$, **$p < 0.01$.

**FIG. 10** shows learning performance on the Morris water maze (MWM). A, escape latency. Mice administered 7C-4MAQ ("N-MCQ") (n=10) exhibited decreased escape latencies compared to vehicle controls (Vehicle, n=10; $p < 0.05$, group effect; $p < 0.01$, N-MCQ×Vehicle), and were indistinguishable from sham mice (Sham, n = 14). After the 5th day of MWM testing, the submerged platform was made visible and all difference between groups disappeared. B, escape latency. Animals administered CQ (n = 10) had a performance profile similar to their N-MCQ counterparts ($p < 0.05$, group effect; $p < 0.001$, CQ×Vehicle). C, probe trial. Mice administered N-MCQ spent significantly more time in the target quadrant compared to all other treatment groups ($p < 0.05$, group effect). D, swim speed. No differences in swim speed were observed by treatment group. Values represent averages ±SEM. *$p < 0.05$, **$p < 0.01$, ***$p < 0.001$.5, $p < 0.01$; Sham×Vehicle, n = 9, $p < 0.05$).Values represent averages ±SEM. *$p < 0.05$, **$p < 0.01$.

**FIG. 11** shows that aminoquinolines decrease microgliosis and astrocytosis in WM tracts of BCAS mice. A, representative Iba-1 and GFAP staining of the medial CC (bregma = 0 mm) at day three. B, Iba-1 immuno-positive cell density in multiple WM tracts at day three and 32. Vehicle controls (n = 9) had a significantly higher density of Iba-1 positive cells in multiple WM tracts at day three and 32 compared to other treatment groups (n = 5). C, GFAP immuno-positive cell density. Vehicle controls had a significantly higher density of GFAP positive cells in the CC at day three and 32 compared to all other treatment groups. Values represent averages ±SEM. *$p < 0.05$, **$p < 0.01$, ***$p < 0.001$. positive cells in the CC at day three and 32 compared to all other treatment groups. Values represent averages ±SEM. *$p < 0.05$, **$p < 0.01$, ***$p < 0.001$.

**FIG. 12** shows that inhibition of QR2 decreases oxidative stress in WM tracts of BCAS mice. The vehicle group (n = 9) exhibited significantly higher 8-OhdG staining density than all other treatment groups (CQ, n = 8; N-MCQ, n = 7; Vehicle, n = 9) on day 32 in the IC, and a higher density than sham and N-MCQ groups in the CC on day three. Values represent averages ±SEM. *$p < 0.05$, **$p < 0.01$, ***$p < 0.001$.

## DETAILED DESCRIPTION

Provided herein are compounds for use in treating acute neural injury, vascular dementia or CNS lupus. In some embodiments, quinoline and quinazoline derivatives useful in inhibiting quinone reductase 2 (QR2) are provided for such treatment.

As used herein in the description of the invention and the appended claims, the singular forms "a," "an" and "the" are intended to include the plural forms as well, unless the context clearly indicates otherwise. As used herein in the description of the invention and the appended claims, the singular forms "a," "an" and "the" are intended to include the plural forms as well, unless the context clearly indicates otherwise. Furthermore, the terms "about" and "approximately" as used herein when referring to a measurable value such as an amount of a compound, dose, time, temperature, and

the like, is meant to encompass variations of 20%, 10%, 5%, 1%, 0.5%, or even 0.1% of the specified amount. Also, as used herein, "and/or" and "/" refer to and encompass any and all possible combinations of one or more of the associated listed items, as well as the lack of combinations when interpreted in the alternative ("or").

## I. Definitions

The following definitions are used herein.

As known in the art, "H" refers to a hydrogen atom. "C" refers to a carbon atom. "N" refers to a nitrogen atom. "O" refers to an oxygen atom.

"Halo" refers to F, Cl, Br or I. "Cl" is chloro, "I" is iodo, "F" is fluoro, and "Br" is bromo.

An "acyl" is a group -C(O)R, where R is a suitable substituent (for example, an acetyl group, a propionyl group, a butyroyl group, a benzoyl group, or an alkylbenzoyl group).

"Alkyl," as used herein, refers to a straight or branched chain saturated hydrocarbon containing from 1 or 2 to 10 or 20 or more carbon atoms (e.g., C2, C3, C4, C5, C6, C7, C8, C9, C10, C 11, C12, C13, C14, C15, etc.). Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, and the like. In some embodiments, the alkyl is a "lower alkyl" having from 1 to 3, 4, or 5 carbon atoms.

"Alkenyl" as used herein is a straight or branched chain unsaturated hydrocarbon group having one or more double bonds.

"Alkynyl" as used herein is a straight or branched chain unsaturated hydrocarbon group having one or more triple bonds.

"Amino" is the group $-NH_2$. An "amide" or "amido" as used herein refers to an organic functional group having a carbonyl group (C=O) linked to a nitrogen atom (N). "Alkylamino" refers to an alkyl group, as defined herein, appended to the parent molecule through a nitrogen atom (-NH-).

"Alkoxy," as used herein, refers to an alkyl group, as defined herein, appended to the parent molecule through an oxygen atom (-O-). Representative examples of alkoxy include, but are not limited to, methoxy, ethoxy, propoxy, 2-propoxy, butoxy, tert-butoxy, pentyloxy, hexyloxy and the like.

"Aryl" as used herein refers to a ring system having one or more aromatic rings. Representative examples of aryl include azulenyl, indanyl, indenyl, naphthyl, phenyl, tetrahydronaphthyl, and the like. The aryl groups of this invention can be substituted with 1, 2, 3, 4, or 5 substituents independently selected from alkenyl, alkenyloxy, alkoxy, alkoxyalkoxy, alkoxycarbonyl, alkyl, alkylcarbonyl, alkylcarbonyloxy, alkylsulfinyl, alkylsulfonyl, alkylthio, alkynyl, aryl, aryloxy, azido, arylalkoxy, arylalkyl, aryloxy, carboxy, cyano, formyl, halo, haloalkyl, haloalkoxy, hydroxy, hydroxyalkyl, mercapto, nitro, sulfamyl, sulfo, sulfonate, NR'R" (wherein, R' and R" are independently selected from hydrogen, alkyl, alkylcarbonyl, aryl, arylalkyl and formyl), and -C(O)NR'R" (wherein R' and R" are independently selected from hydrogen, alkyl, alkylcarbonyl, aryl, arylalkyl, and formyl).

"Cycloalkyl" refers to a monocyclic or fused polycyclic C3 to C10 saturated hydrocarbon groups. "Heterocycloalkyl" refers to a cycloalkyl group in which one or more carbon atoms have been replaced with atoms independently selected from the group consisting of: O, N, and S.

"Haloalkyl," as used herein, a refers to a straight or branched chain hydrocarbon containing from 1 or 2 to 10 or 20 or more carbon atoms *(e.g.,* C2, C3, C4, C5, C6, C7, C8, C9, C10, C11, C12, C13, C14, C15, etc.) in which at least one of the hydrogen atoms have been replaced with halo (F, Cl, Br or I). Representative examples of "haloalkyl" include, but are not limited to, fluoroalkyl (e.g., fluoromethyl ($-CH_2F$), difluoromethyl ($-CHF_2$), or trifluoromethyl ($-CF_3$)).

"Heterocyclo," as used herein, refers to a monocyclic, bicyclic or tricyclic ring system containing at least one heteroatom selected from O, N, and S. Monocyclic heterocycle ring systems are exemplified by any 5 or 6 member ring containing 1, 2, 3, or 4 heteroatoms independently selected from the group consisting of O, N, and S. The 5 member ring has from 0 to 2 double bonds, and the 6 member ring has from 0 to 3 double bonds. Representative examples of monocyclic ring systems include, but are not limited to, azetidine, azepine, aziridine, diazepine, 1,3-dioxolane, dioxane, dithiane, furan, imidazole, imidazoline, imidazolidine, isothiazole, isothiazoline, isothiazolidine, isoxazole, isoxazoline, isoxazolidine, morpholine, oxadiazole, oxadiazoline, oxadiazolidine, oxazole, oxazoline, oxazolidine, piperazine, piperidine, pyran, pyrazine, pyrazole, pyrazoline, pyrazolidine, pyridine, pyrimidine, pyridazine, pyrrole, pyrroline, pyrrolidine, tetrahydrofuran, tetrahydrothiophene, tetrazine, tetrazole, thiadiazole, thiadiazoline, thiadiazolidine, thiazole, thiazoline, thiazolidine, thiophene, thiomorpholine, thiomorpholine sulfone, sulfoxide, thiopyran, triazine, triazole, trithiane, and the like. Bicyclic ring systems are exemplified by any of the above monocyclic ring systems fused to an aryl group as defined herein, a cycloalkyl group as defined herein, or another monocyclic ring system as defined herein. Representative examples of bicyclic ring systems include but are not limited to, for example, benzimidazole, benzothiazole, benzothiadiazole, benzothiophene, benzoxadiazole, benzoxazole, benzofuran, benzopyran, benzothiopyran, benzodioxine, 1,3-benzodioxole, cinnoline, indazole, indole, indoline, indolizine, naphthyridine, isobenzofuran, isobenzothiophene, isoindole, isoindoline, isoquinoline, phthalazine, pyranopyridine, quinoline, quinolizine, quinoxaline, quinazoline, tetrahydroisoquinoline, tetrahydroquinoline, thiopyranopyridine, and the like. Examples of nitrogen-containing heterocyclo include, but are not limited to, pyrrolidinyl, piperidinyl, piperazinyl, morpholinyl, etc.

"Heteroaryl" means a cyclic, aromatic hydrocarbon in which one or more carbon atoms have been replaced with atoms independently selected from the group consisting of O, N, and S. Examples of heteroaryl groups include pyridyl, pyrimidinyl, imidazolyl, thienyl, furyl, pyrazinyl, pyrrolyl, pyranyl, isobenzofuranyl, chromenyl, xanthenyl, indolyl, isoindolyl, indolizinyl, triazolyl, pyridazinyl, indazolyl, purinyl, quinolizinyl, isoquinolyl, quinolyl, phthalazinyl, naphthyridinyl, quinoxalinyl, isothiazolyl, and benzo[b]thienyl. Preferred heteroaryl groups are five and six membered rings and contain from one to three heteroatoms independently selected from the group consisting of O, N, and S. The heteroaryl group, including each heteroatom, can be unsubstituted or substituted with from 1 to 4 suitable substituents, as chemically feasible. For example, the heteroatom S may be substituted with one or two oxo groups, which may be shown as =O. Examples of nitrogen-containing heteroaryls include, but are not limited to, pyridinyl, pyrimidinyl, pyrazinyl, pyridazinyl, imidazolyl, pyrrolyl, pyrazolyl, thiazolyl, triazolyl, isothiazolyl, indolyl, benzimidazolyl, benzoxazolyl, quinolinyl, isoquinolinyl, quinazolinyl, acridinyl, carbazole, azepinyl, 1,4-diazepinyl, purinyl, pteridinyl, phthalazinyl, etc.

"Hydroxyl" and "hydroxy" refer to the group -OH.

"Nitrile" refers to the group -CN.

"Nitro" refers to the group $-NO_2$.

A "sulfone" refers to a sulfonyl functional group, $-SO_2R$, wherein R is any covalently linked atom or atoms.

A "sulfoxide" refers to the group -S(O)R, wherein R is any covalently linked atom or atoms.

A "thiol" or "mercapto" refers to the group -SH or to its tautomer =S.

A "ureido" refers to the group $-NHCONH_2$. A "thioureido" refers to the group $-NHCSNH_2$.

A "pharmaceutically acceptable salt" is a salt that retains the biological effectiveness of the free acids and bases of a specified compound and that is not biologically or otherwise undesirable. Examples of pharmaceutically acceptable salts include, but are not limited to, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, $\gamma$-hydroxybutyrates, glycollates, tartrates, methane-sulfonates, propanesulfonates, naphthalene-1-sulfonates, naphthalene-2-sulfonates, and mandelates.

A "prodrug" as known in the art is a compound that can be converted under physiological conditions or by solvolysis or metabolically to a specified compound that is pharmaceutically active. A thorough discussion is provided in T. Higuchi and V. Stella, Prodrugs as Novel delivery Systems, Vol. 14 of the A.C.S. Symposium Series and in Edward B. Roche, ed., Bioreversible Carriers in Drug Design, American Pharmaceutical Association and Pergamon Press, 1987. *See also* US Patent No. 6,680,299. Examples include a prodrug that is metabolized *in vivo* by a subject to an active compound as described herein, wherein the prodrug is an ester of an alcohol or carboxylic acid group, if such a group is present in the compound; an acetal or ketal of an alcohol group, if such a group is present in the compound; an N-Mannich base or an imine of an amine group, if such a group is present in the compound; or a Schiff base, oxime, acetal, enol ester, oxazolidine, or thiazolidine of a carbonyl group, if such a group is present in the compound, such as described in US Patent No. 6,680,324 and US Patent No. 6,680,322.

As understood in the art, the term "optionally substituted" indicates that the specified group is either unsubstituted, or substituted by one or more suitable substituents. A "substituent" that is "substituted" is a group which takes the place of a hydrogen atom on the parent organic molecule.

## II. Active Compounds

Provided herein as an active compound according to some embodiments is a compound of **Formula I:**

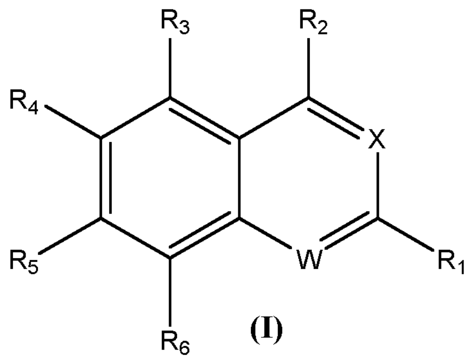

(I)

wherein:

WisN;
X is $CR_{14}$;
$R_1$ is H or trifluoromethyl;
$R_2$ is $NR_7R_8$, $OR_{11}$, $SR_{12}$, or alkyl;
$R_3$ is H or $OR_{13}$;
$R_4$ is H or methoxy;
$R_5$ is H, Cl, or trifluoromethyl;
$R_6$ is H or trifluoromethyl;
$R_7$ is H, $C_{1-5}$ alkyl, heteroarylalkyl, cycloalkyl, heterocycloalkyl, heterocyclo, aryl, heteroaryl, ureido, thioureido, alkenyl, alkynyl, amido, amino, alkoxy, alkylamino, alkylphosphonate, alkylnitrile, alkylhalo, or alkylhalo optionally substituted with $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo;
$R_8$ is H, $C_{1-5}$ alkyl, cycloalkyl, heterocycloalkyl, aryl, heteroaryl, ureido, thioureido, alkenyl, alkynyl, amido, amino, alkoxy, alkylamino, alkylphosphonate, alkylnitrile, alkylhalo or alkylhalo optionally substituted with $C_{1-5}$ alkyl, cycloalkyl, cycloalkenyl, heterocycloalkyl, heteroaryl, alkenyl, alkynyl, amido, alkoxy, alkylhydroxy, halo, hydroxyl, carboxylate, alkylcarboxylate, acylazido, sulfonamide or alkylhalo;
$R_9$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, aryl, heteroaryl or alkylamino;
$R_{10}$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, heteroaryl, or alkylamino;
$R_{11}$ is alkyl, aryl or heteroaryl optionally substituted with alkyl, haloalkyl, aryl or heteroaryl;
$R_{12}$ is alkyl, aryl or heteroaryl optionally substituted with alkyl, haloalkyl, aryl or heteroaryl;
$R_{13}$ is alkyl or aryl optionally substituted with alkyl or haloalkyl; and
$R_{14}$ is H or aryl;

or a pharmaceutically acceptable salt thereof.

In some embodiments of Formula I, $R_1$ is H. In some embodiments of Formula I, $R_2$ is $NR_7R_8$. In some embodiments of Formula I, $R_3$ is H. In some embodiments of Formula I, $R_4$ is H. In some embodiments of Formula I, $R_5$ is Cl. In some embodiments of Formula I, $R_6$ is H. In some embodiments of Formula I, $R_7$ is H. In some embodiments of Formula I, $R_8$ is $C_{1-5}$ alkyl substituted with heteroaryl. In some embodiments of Formula I, $R_{14}$ is H.

In some embodiments of **Formula I,** the compound is a compound of **Formula I(a):**

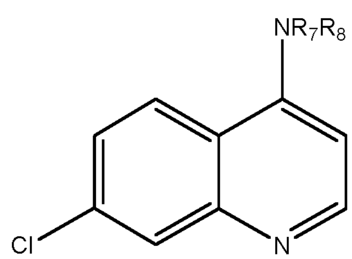

I(a)

wherein $R_7$ and $R_8$ are each independently H or $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, e.g., $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo).

In some embodiments of Formula I(a), one of $R_7$ and $R_8$ is hydrogen, and the other is $C_{1-5}$ alkyl, wherein said $C_{1-5}$ alkyl is optionally substituted with cycloalkyl, heterocycloalkyl, heterocyclo, aryl, or heteroaryl (which may each be further substituted with any suitable substituent, e.g., $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo).

In some embodiments of **Formula I,** the compound is:

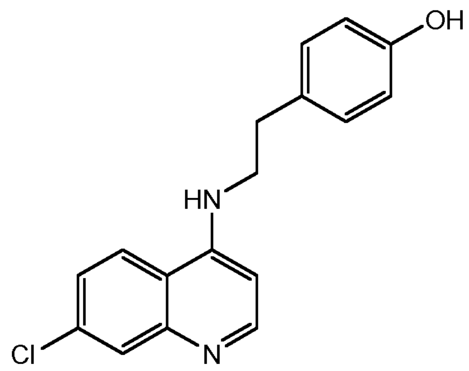

4-{2-[(7-chloroquinolin-4-yl)amino]ethyl}phenol,

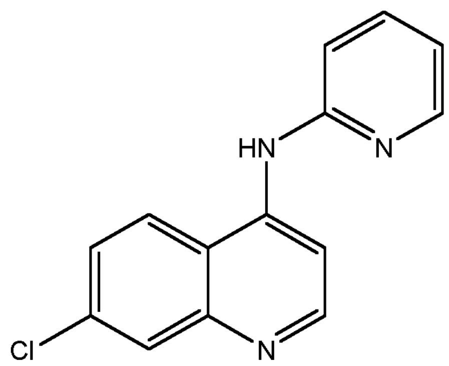

7-chloro-N-(pyridin-2-yl)quinolin-4-amine ,

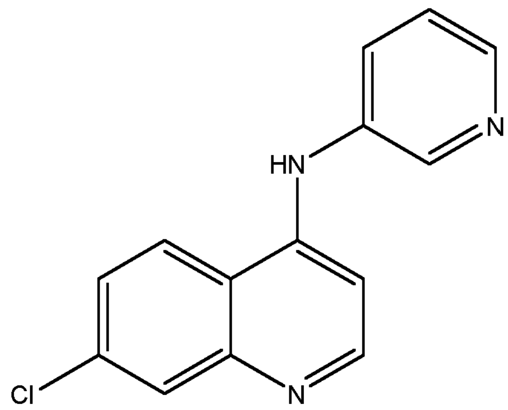

7-chloro-N-(pyridin-3-yl)quinolin-4-amine , or

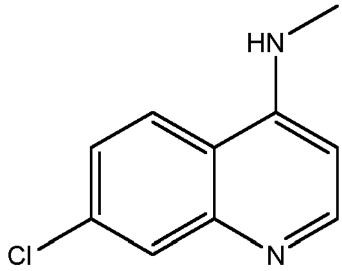

7-chloro-N-methylquinolin-4-amine
or a pharmaceutically acceptable salt thereof.

In some embodiments of **Formula I,** the compound is:

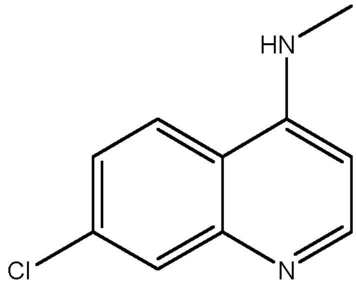

7-chloro-N-methylquinolin-4-amine ,
or a pharmaceutically acceptable salt thereof.

Further provided herein as an active compound is a compound of **Formula** II:

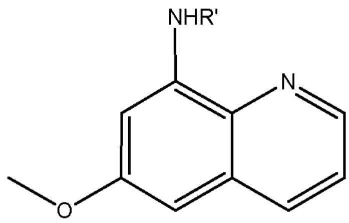

II

wherein R' is selected from the group consisting of pyridin-2-ylmethyl, pyridin-3-ylmethyl, 1-benzylpiperidin-4-yl, 4-cyano-2,2-diethylbutyl, 2-chlorocyclopentyl, 4-(diethylamino)butan-2-yl, 1-(furan-2-yl)ethyl, 1-cyclopropylethyl, 1-ethylpiperidin-4-yl, 5-amino-2,2-diethylpentyl, and 2-(diethylphosphoryl)-1-methylethyl,
or a pharmaceutically acceptable salt thereof.

In some embodiments of **Formula II,** the compound is:

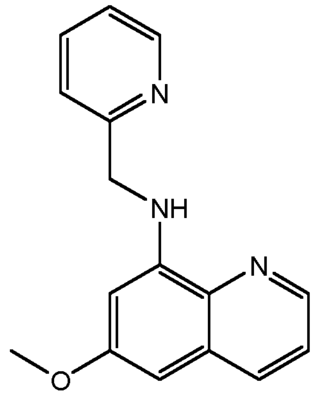

6-methoxy-N-(pyridin-2-ylmethyl)quinolin-8-amine , or

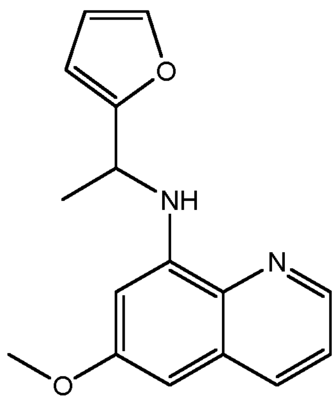

N-[1-(furan-2-yl)ethyl]-6-methoxyquinolin-8-amine ,
or a pharmaceutically acceptable salt thereof.

Further active compounds may be found in U.S. Patent Application Publication No. 2006/0074105 to Ware, Jr., et al.. The compounds may be prepared according to known methods such as those described in Egan et al., J. Med. Chem. 2000, 43:283-291; Stocks et al., J. Med. Chem. 2002, 45:4975-4983; or by methods described herein in the examples provided below.

In some embodiments of the above compound of Formula I or Formula II, the compound has a positive log D value at approximately pH 4 to pH 5.

Unless otherwise stated, structures depicted herein are also meant to include all enantiomeric, diastereomeric, and geometric (or conformational) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Tautomeric forms include keto-enol tautomers of a compound. In addition, unless otherwise stated, all rotamer forms of the compounds of the invention are within the scope of the invention. Unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a $^{13}C$- or $^{14}C$-enriched carbon are within the scope of this invention. Such compounds are useful, for example, as analytical tools or probes in biological assays.

**III. Methods of Use**

As noted above, active compounds as taught herein are useful for treatment of acute neural injury, vascular dementia or CNS lupus.

Acute neural injury includes, but is not limited to, traumatic brain injury and non-traumatic acute brain injury. Traumatic brain injury, as known in the art, is damage and/or dysfunction of the brain caused by a single or repetitive external mechanical force, such as blunt force or sheer force from sudden acceleration or deceleration. Traumatic brain injury includes, but is not limited to, concussion, contusion, and hemorrhage, including parenchymal, subdural, epidural,

and subarachnoid hemorrhage. Other acute neural injuries include insult from hypoxic or ischemic brain injury, e.g., from arterial stroke (focal, global), venous infarction, infection, perioperative cerebral injury, etc.

Vascular dementia is dementia or cognitive deficit caused by acute cerebrovascular compromise, often associated with multiple cerebrovascular events such as strokes.

Central nervous system lupus (CNS lupus) refers to neurological and/or behavioral clinical syndromes in subjects with systemic lupus erythematosus (SLE). CNS lupus may present clinically as acute confusion, fatigue, headache, subtle cognitive impairment, delirium, coma, dementia, sensory/motor/autonomic deficits, and/or seizures (the latter which occur more frequently in lupus patients than the general population). CNS lupus may also present as psychological disorders such as depression, mania, and/or psychosis. More focal neurological deficits are also possible and may occur secondary to lupus-related embolic, thrombotic or vasculitic infarction of brain and spine as well as cranial neuropathies. Pathophysiological mechanisms of CNS lupus may include cerebritis, transverse myelitis, neuritis and stroke (embolic, thrombotic, or vasculitic) of the brain or spine.

The term "treat" as used herein refers to any type of treatment that imparts a benefit to a subject afflicted with or at risk of an injury, disease or disorder (e.g., improvement or decreased risk of developing one or more symptoms such as cognitive dysfunction and/or motor dysfunction), delay in the progression of the injury or symptoms, etc.

The present invention is primarily concerned with the treatment of human subjects, but the invention may also be carried out on animal subjects, particularly mammalian subjects such as mice, rats, dogs, cats, livestock and horses for veterinary purposes, and/or for drug screening and/or drug development purposes.

## IV. Formulations

In some embodiments, active compound(s) may be provided in a pharmaceutically acceptable carrier. Carriers should be acceptable in that they are compatible with any other ingredients of the formulation and not harmful to the recipient thereof. In some embodiments, the pharmaceutically acceptable carrier is a sterile (e.g., endotoxin-free or pyrogen-free water, or endotoxin-free or pyrogen-free water saline).

Formulations of the present invention may include short-term, rapid-onset, rapid-offset, controlled release, sustained release, delayed release, and pulsatile release formulations, providing the formulations achieve administration of a compound as described herein. See Remington's Pharmaceutical Sciences (18th ed.; Mack Publishing Company, Eaton, Pa., 1990).

Pharmaceutical formulations according to the present invention may be suitable for various modes of delivery, including oral, parenteral (including intravenous, intramuscular, subcutaneous, intradermal, and transdermal), topical (including dermal, buccal, and sublingual), and rectal administration.

Examples of suitable dosage unit forms in accordance with this invention are tablets, capsules, orally administered liquid preparations in suitable liquid vehicles, sterile preparations in suitable liquid vehicles for intramuscular and intravenous administration, suppositories, and sterile dry preparations for the extemporaneous preparation of sterile injectable preparations in a suitable pharmaceutically acceptable carrier. Suitable solid diluents or carriers for the solid oral pharmaceutical dosage unit forms may be selected from the group consisting of lipids, carbohydrates, proteins and mineral solids; for example, starch, sucrose, kaolin, dicalcium phosphate, gelatin, acacia, corn starch, talc, and the like. Capsules, both hard and soft, may be formulated with suitable diluents and excipients; for example, edible oils, talc, calcium carbonate, and the like, and also, calcium stearate. Liquid preparations for oral administration may be prepared in water or aqueous solutions containing suspending agents; for example, sodium carboxymethylcellulose, methylcellulose, acacia, polyvinyl pyrrolidone, polyvinyl alcohol and the like. In some embodiments, preservatives may be included, for example, parabens, chlorobutanol, benzyl alcohol phenol, and the like. *See* U.S. Patent No. 4,159,331 to McCall.

The amount of active compound(s) administered for therapeutic treatment may depend on the age, weight, and condition of the patient as determined by a physician. In some embodiments, the administration and/or pharmaceutical dosage unit form may provide from about 0.05 mg to about 100 mg of the active compound(s) per dosage. In some embodiments, active compound(s) are provided an amount of from about 1 microgram per kg to about 1 g per kg of body weight of the recipient, or 10 micrograms to 100 mg per kg, or 0.1 mg to 50 mg per kg of body weight.

The present invention is explained in greater detail in the following non-limiting examples.

EXAMPLES

### Example 1: Development of non-lysosomotropic aminoquinoline inhibitors of QR2

Chloroquine and hydroxychloroquine are lysosomotropic drugs that accumulate preferentially in cellular lysosomes. Their structures are:

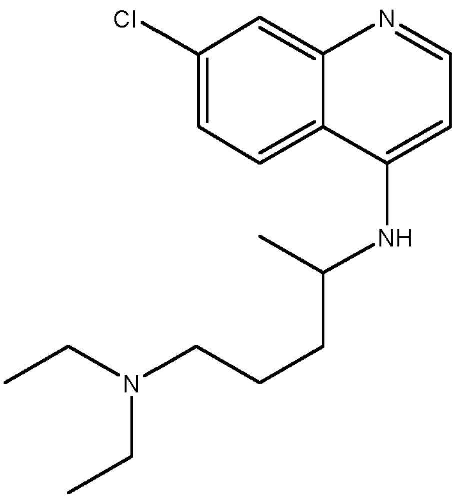

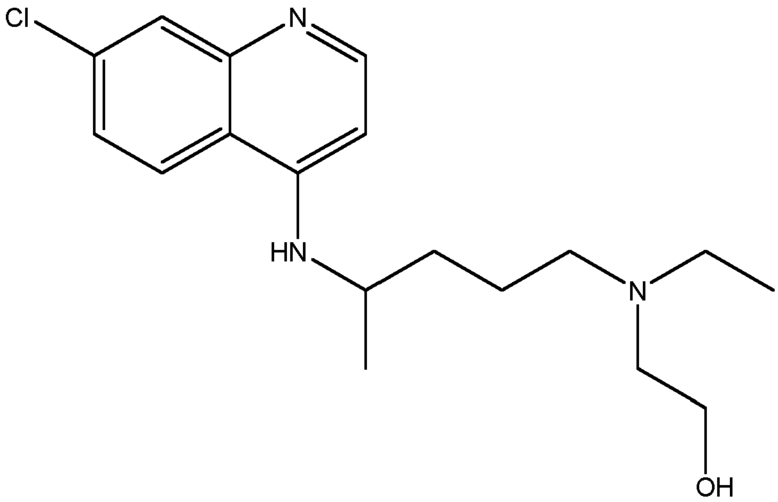

chloroquine hydroxychloroquine

For chloroquine, the pKa of the tertiary amine nitrogen is 10.32 and that of the quinoline nitrogen is 7.29. At acidic lysomal pHs between 4 and 5.5, nearly 100% of chloroquine is therefore doubly protonated, rendering the molecule with a 2+ charge that makes the molecule strongly hydrophilic, membrane impermeable, and thus trapped in the acidic organelle.

A quantitative treatment of this trapping phenomenon can be obtained by examining the octanol-water distribution coefficient, log D, of a drug, which depicts the relative partition properties for all forms of a compound at different pH. Compounds with positive logD for a given pH are relatively lipophilic and more membrane permeable, whereas compounds with a negative logD are hydrophilic and less membrane permeable.

**FIG. 1A** shows the logD of chloroquine, and **FIG. 1B** shows the logD of hydroxychloroquine. The shaded regions in the figures represent the potential range of lysosomal pH encountered *in vivo.* At lysosomal pH, the logD of both chloroquine and hydroxychloroquine are substantially negative, reflecting the accumulated charge of these molecules and their loss of membrane permeability.

A chemoproteomic strategy was used to generate a chemical library of 4-aminoquinoline scaffolds with selectivity for QR2. Using an array of chemi-informatics tools, we have mined this library *in silico* and have identified aminoquinoline derivatives with nanomolar to micromolar inhibition of QR2 that also possess chemical properties avoiding lysosomal accumulation, thereby addressing the mechanism responsible for CQ/HQ's most common toxicities.

Example compounds as provided below, and their respective log D predictions are shown in **FIGS. 2A-2E.** Note that regardless of acidity, log D values remain positive (> 0.5) for each compound, indicating that these molecules will retain lipophilicity (and thus membrane permeability) at lysosomal pH (between pH 4 and 5).

**Example Compound A** (logD shown in **FIG. 2A):**

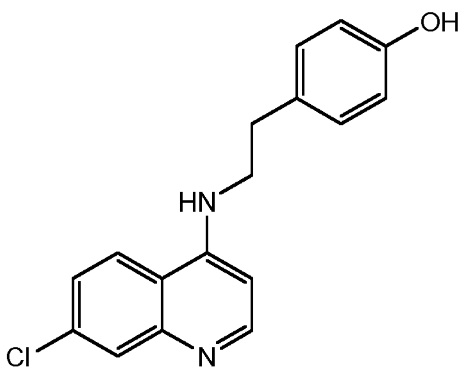

4-{2-[(7-chloroquinolin-4-yl)amino]ethyl}phenol

**Example Compound B** (logD shown in **FIG. 2B**):

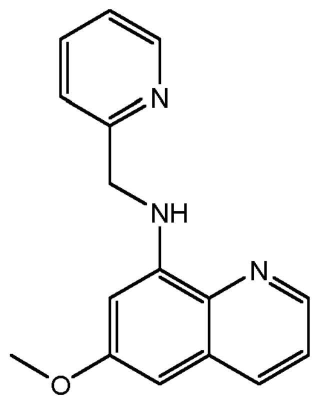

6-methoxy-N-(pyridin-2-ylmethyl)quinolin-8-amine

**Example Compound** C (logD shown in **FIG. 2C):**

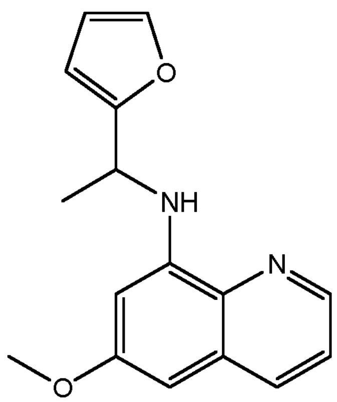

N-[1-(furan-2-yl)ethyl]-6-methoxyquinolin-8-amine

**Example Compound D** (logD shown in **FIG. 2D):**

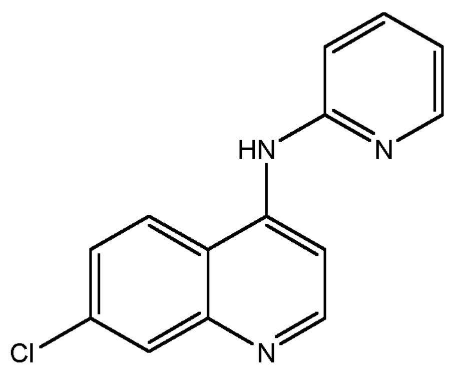

7-chloro-N-(pyridin-2-yl)quinolin-4-amine

**Example Compound E** (logD shown in **FIG. 2E):**

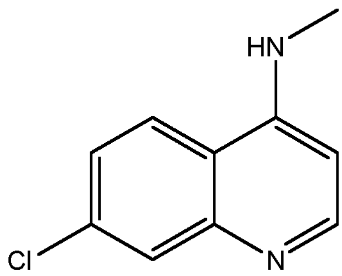

7-chloro-N-methylquinolin-4-amine

**Example Compound F:**

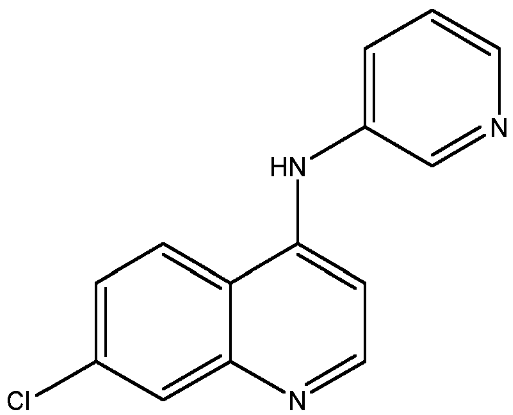

7-chloro-N-(pyridin-3-yl)quinolin-4-amine

**Table 1** below presents additional estimates of drug-likeness of these non-lysosomotropic 4-aminoquinolines as compared to chloroquine (CQ). Lipophilic efficiency, LiPE (also known as ligand lipophilicity efficiency) is a drug design and discovery parameter linking potency with lipophilicity. LiPE is defined as the $pIC_{50}$ (-log $IC_{50}$) minus the calculated log P, clog P:

$$\text{LiPE} = \text{pIC}_{50} - \text{clogP}$$

LiPE is used to estimate *in vivo* drug specificity, with higher values predictive of increased potency and decreased probability for unwanted or off-target interactions. LiPE for many of the disclosed 4-aminoquinoline QR2 inhibitors are higher than CQ, therefore predicting a better toxicity profile than CQ has, independent of the substantially reduced toxicity anticipated through elimination of lysosomotropism.

**Table 1:** Empiric and calculated parameters of drug-likeness for 4-aminoquinoline inhibitors of QR2.

| **Compound** | **$IC_{50}$ (μM) mean +/- SD** | **clog P** | **LiPE ($pIC_{50}$ - clogP)** |
|---|---|---|---|
| chloroquine | 1.13 +/-0.8 | 3.93 | 2.01 |
| 4-{2-[(7-chloroquinolin-4-yl)amino]ethyl}phenol | 2.7 +/- 0.2 | 3.92 | 1.65 |
| 6-methoxy-N-(pyridin-2-ylmethyl)quinolin-8-amine | 0.47 +/- 0.26 | 2.03 | 4.30 |

(continued)

| Compound | $IC_{50}$ (μM) mean +/- SD | clog P | LiPE ($pIC_{50}$ - clogP) |
|---|---|---|---|
| N-[1-(furan-2-yl)ethyl]-6-methoxyquinolin-8-amine | 0.85 +/- 0.15 | 2.65 | 3.42 |
| 7-chloro-N-(pyridin-2-yl)quinolin-4-amine | 0.55 +/-0.03 | 3.55 | 2.71 |
| 7-chloro-N-methylquinolin-4-amine | 0.13 +/- 0.03 | 2.21 | 4.67 |

**Example 2: 7-Chloro Compound Synthesis and Characterization**

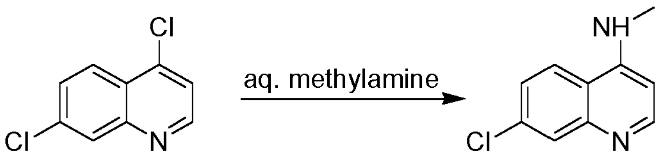


A suspension of 4,7-dichloroquinoline (2.0g, 10.2 mmol) in aqueous methylamine (40% 20 mL 260 mmol, 26 eq.) was heated in a microwave vessel at 90 °C (initial power setting of 150W) for 2h. Analysis of the reaction mixture by TLC (2% MeOH in $CH_2Cl_2$) indicated complete consumption of starting material. The reaction mixture was diluted with $H_2O$ (100 mL) and insoluble were collected at the vacuum. The filter cake was washed with $H_2O$ and dried in vacuo giving the pure product as a white micro crystalline solid (1.8g, 92%). $^1H$ NMR (DMSO-$d_6$, 300 MHz) δ 8.40 (d, J = 5.1 Hz, 1H), 8.16 (d, J = 9.0 Hz, 1H), 7.77 (s, 1H), 6.38 (d, J = 5.4 Hz, 1H), 2.86 (d, J = 5.4 Hz, 3H). ESIMS: *m/z* = 193 [$(M+H)^+$].

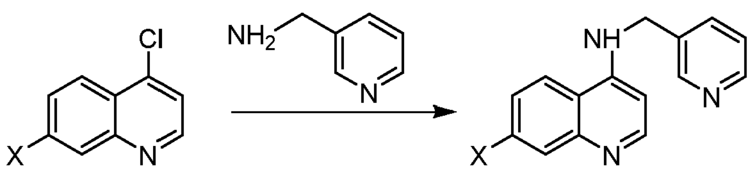

**General procedure for 7-substituted-4-(pyridin-3-yl)-methylaminoquinolines.** A mixture of the 7-substituted-4-chloroquinoline (5.1 mmol), 3-aminomethyl pyridine (0.70 g, 6.2 mmol, 1.2 eq.) and 1-butanol (5 mL) were heated in a sealed heavy walled pressure vessel (12 mL) at 130 °C (bath temperature) for 24h. The vessel was cooled to room temperature and the contents were diluted into $Et_2O$ (150 mL). Insolubles were removed at the vacuum. The filter cake was dissolved in a minimum amount of MeOH and the resulting solution was added to silica gel (~3g). The mixture was concentrated to dryness under reduced pressure. Flash column chromatography (RediSepRf $SiO_2$ (40 g), 100% $CH_2Cl_2$→75% (90:10, $CH_2Cl_2$:MeOH containing 10% $NH_3$) gave the desired products.

X = Cl (white solid, 0.92 g, 67%).

$^1H$ NMR (DMSO-$d_6$, 400 MHz) δ 8.61 (s, 1H), 8.42 (s, 1H), 8.27 (m, 2H), 8.00 (s, 1H), 7.75 (m, 2H), 7.46 (d, J = 8.8 Hz, 1H), 7.31 (m, 3H), 6.39 (d, J = 5.4 Hz, 1H), 4.55 (d, J = 5.4 Hz, 2H). ESIMS: *m/z* = 270 [$(M+H)^+$].

**Example 3: Evidence for the protective role of QR2 inhibition in cerebral infarction and therapeutic effectiveness of non-lysosomotropic inhibitors of QR2.**

The neuroprotective efficacy of chloroquine (CQ) was demonstrated in a mouse model of transient middle cerebral artery (MCA) occlusion. Post-mortem histological assessments at 72 hours show that a single i.p. administration of CQ (25 mg/kg) 90 minutes after ischemia onset results in a 55% reduction in overall stroke volume, with corresponding reduction in stroke evolution between 4 and 24 hours as measured by diffusion weighted magnetic resonance imaging (DW-MRI), and improvement in neurological score and motor function, as shown in **FIGS. 3A-3C.**

Also tested was the neuroprotective efficacy of the non-lysosomtropic QR2-selective 4-aminoquinoline, 7-chloro-N-methylquinolin-4-amine (7C-4MAQ, Example Compound E shown above) in the same model. 7-chloro-N-methylquinolin-4-amine results in strikingly significant neuroprotection in this animal model, with reductions in cortical stroke volumes nearly 2x those seen after CQ administration when comparing at an equivalent single, acute phase dose

(25 mg/kg) **(FIG. 3C).**

It is worth noting that the 25 mg/kg dose remains more than 20 times lower than the $LD_{50}$ determined for this compound. Finally, we compared QR2 null mice to their littermate controls in the same MCA occlusion/reperfusion model, as also shown in **FIG. 3C.**

### Example 4: Traumatic Brain Injury (TBI)

The neuroprotective potential of QR2 inhibition was investigated in a murine TBI model of diffuse closed head injury (Laskowitz et al., "Neuroprotective pentapeptide CN-105 is associated with reduced sterile inflammation and improved functional outcomes in a traumatic brain injury murine model." Sci. Rep. 2017 Apr 21;7:46461). As shown in **FIG. 4,** marked improvement following TBI was found in both neurocognitive and neuro-motor function assessment. A single 25 mg/kg administration of 7-chloro-N-methylquinolin-4-amine (7C-4MAQ, Example Compound E shown above) resulted in a 20% improvement over vehicle in rotorod latency (n=12/gp), and more significantly, a 62% improvement in Morris water maze performance, even after 1 month following injury (n=12/gp).

As in the stroke model, nearly identical trends are observed when comparing 7C-4MAQ to CQ, and QR2 null mice to their littermate controls.

### Example 5: Intracranial hemorrhage (ICH)

QR2 inhibition was investigated in a mouse model of intracranial hemorrhagic injury (Lei et al., "Neuroprotective pentapeptide CN-105 improves functional and histological outcomes in a murine model of intracerebral hemorrhage." Sci. Rep. 2016 Oct 7;6:34834). In this study, we investigated 7C-4MAQ, chloroquine (CQ), and an 8-aminoquinonline, primaquine, with the goal of testing the efficacy of the Example Compound while also exploring further the molecular mechanisms of therapeutic action.

It has been previously reported that aminoquinolines such as CQ and hydroxychloroquine inhibit the second half of the QR2 reaction, whereas other quinolines such as primaquine inhibit the first half. In this particular ICH model **(FIG. 5),** it is noted that previous neuroprotective interventions have only shown statistically discernable therapeutic efficacy at the histological and molecular but not behavioral level. In our experiments, CQ therapy also resulted in a non-significant 14% (p=0.3) improvement in motor function (rotorod assessment). However, 7C-4MAQ administration resulted in a statistically significant 21% improvement (p=0.013, two-tail *t*, n=21) in behavioral outcome following a single 25 mg/kg i.p. dose (data not shown). Also noteworthy, primaquine, which selectively inhibits only the first stage of the QR2 reaction, resulted in 35% worsening in motor function (p=0.0001) after an equivalent single i.p. dose (data not shown).

### Example 6: Post-operative cognitive deficit

Perioperative cerebral injury (PCI) following major cardiovascular surgery using cardiopulmonary bypass (CPB) and deep hypothermic circulatory arrest (DHCA) remains a significant cause of adverse cerebral outcome. We compared the effects of CQ versus 7C-4MAQ QR2 inhibition on cerebral outcome following cardiopulmonary bypass (CPB)/deep hypothermic circulatory arrest (DHCA) in a well-established rat model originally developed in Dr. Podgoreanu's laboratory at Duke (de Lange et al., "A novel survival model of cardioplegic arrest and cardiopulmonary bypass in rats: a methodology paper," J Cardiothorac Surg. 2008 Aug 19;3:51). Results are shown in **FIGS. 6, 7 and 8.** For this model of CPB/DHCA, fasting adult male Sprague-Dawley rats (10-12 weeks old) were anaesthetized with inhaled isoflurane 2-2.5%, intubated and mechanically ventilated. Cannulas were placed in the tail artery and the right external jugular vein. Animals were then cooled on CPB for 30 minutes, and DHCA was instituted at a pericranial temperature of 16-18 °C. Following 60 minutes of DHCA, CPB was reinitiated, animals were rewarmed for 30 minutes, and separated from CPB at a temperature ≥35.5 °C. MRI was performed on day-1 post-operatively, neurological assessments on day 1 and 2 post-operatively, and animals then sacrificed after day 2.

MRI analysis of preliminary results reveals a 3% decrease in post-operative blood brain barrier permeability as measured by gadolinium chelate in animals treated with chloroquine (CQ) or the Example Compound (7C-4MAQ) compared to their respective control groups (p< 0.05). Animals treated with either CQ or 7C-4MAQ also show fewer apoptotic and necrotic neurons in cortex and hippocampus **(FIG. 6, FIG.** 7). Finally, 7C-4MAQ-treated rats demonstrate significantly improved neurological scores at post-op day 1 and 2 **(FIG. 8).**

### Example 7: Dementia, vascular subtype

Vascular dementia is caused by chronic cerebral hypoperfusion and is characterized clinically by white matter lesions on MRI and a decline in executive function. Recent studies have shown that hippocampal expression of quinone oxidoreductase 2 (QR2) is significantly increased in rat models as well as human patients with dementia, suggesting

QR2 as a possible therapeutic target. We examined the neuroprotective action of chloroquine and 7C-4MAQ in a murine model of vascular dementia. Physiological, cellular, and functional outcomes were determined using a combination of quantitative immunochemistry, MRI, and behavioral tasks including Morris water maze and rotorod.

As shown in **FIG. 9-FIG. 12,** both QR2 inhibitors improved performance on Morris water maze while decreasing astrocytosis, microgliosis, and markers of oxidative stress. Note that in **FIG. 9-FIG. 12**, 7C-4MAQ is referred to using a previous designation, "N-MCG." Despite improvements in functional outcome and cellular inflammatory responses, structural markers of white matter injury were unchanged between treatment and control groups. These results provide evidence of a pathologic role for QR2 in dementia and its potential as a therapeutic target. In addition, the results suggest that functionally relevant neuroprotection occurs through mechanisms independent of those responsible for dementia-associated white matter lesions often characterized on MRI.

## Claims

1. A compound of **Formula I:**

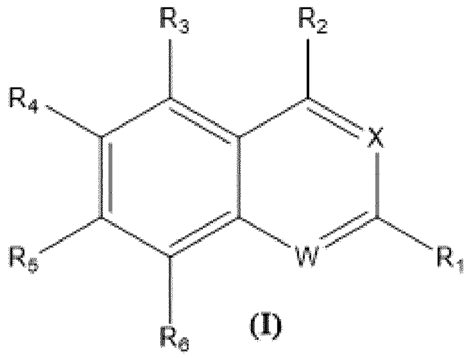

(I)

wherein:

W is N;
X is $CR_{14}$;
$R_1$ is H or trifluoromethyl;
$R_2$ is $NR_7R_8$, $OR_{11}$, $SR_{12}$, or alkyl;
$R_3$ is H or $OR_{13}$;
$R_4$ is H or methoxy;
$R_5$ is H, Cl, or trifluoromethyl;
$R_6$ is H or trifluoromethyl;
$R_7$ is H, $C_{1-5}$ alkyl, heteroarylalkyl, cycloalkyl, heterocycloalkyl, heterocyclo, aryl, heteroaryl, ureido, thioureido, alkenyl, alkynyl, amido, amino, alkoxy, alkylamino, alkylphosphonate, alkylnitrile, or alkylhalo optionally substituted with $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo;
$R_8$ is H, $C_{1-5}$ alkyl, cycloalkyl, heterocycloalkyl, aryl, heteroaryl, ureido, thioureido, alkenyl, alkynyl, amido, amino, alkoxy, alkylamino, alkylphosphonate, alkylnitrile, or alkylhalo optionally substituted with $C_{1-5}$ alkyl, cycloalkyl, cycloalkenyl, heterocycloalkyl, heteroaryl, alkenyl, alkynyl, amido, alkoxy, alkylhydroxy, halo, hydroxyl, carboxylate, alkylcarboxylate, acylazido, sulfonamide or alkylhalo;
$R_9$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, aryl, heteroaryl or alkylamino;
$R_{10}$ is H, O, $C_{1-5}$ alkyl, cycloalkyl, heterocycloalkyl, aryl, heteroaryl, alkylamino, alkylnitrile or alkylphosphonate optionally substituted with $C_{1-5}$ alkyl, cycloalkyl, heterocycloalkyl, heteroaryl, or alkylamino;
$R_{11}$ is alkyl, aryl or heteroaryl optionally substituted with alkyl, haloalkyl, aryl or heteroaryl;
$R_{12}$ is alkyl, aryl or heteroaryl optionally substituted with alkyl, haloalkyl, aryl or heteroaryl;
$R_{13}$ is alkyl or aryl optionally substituted with alkyl or haloalkyl; and
$R_{14}$ is H or aryl;
or a pharmaceutically acceptable salt thereof,
for use in treating acute neural injury, vascular dementia, or central nervous system (CNS) lupus in a subject in need thereof.

2. The compound for use of claim 1, wherein $R_{14}$ is H.

3. The compound for use of any preceding claim, wherein $R_2$ is $NR_7R_8$, $R_7$ is H, and $R_8$ is $C_{1-5}$ alkyl substituted with heteroaryl.

4. The compound for use of any preceding claim, wherein $R_1$, $R_3$, $R_4$, and/or $R_6$ is H.

5. The compound for use of any preceding claim, wherein $R_5$ is Cl.

6. A compound of **Formula I(a):**

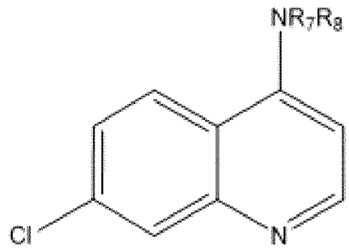

I(a)

wherein $R_7$ and $R_8$ are each independently H or $C_{1-5}$ alkyl, wherein said $C_{1-5}$ alkyl is optionally substituted with cycloalkyl, heterocycloalkyl, heterocyclo, aryl, or heteroaryl (which may be further substituted with $C_{1-5}$ alkyl, cycloalkyl, cycloalkenyl, heterocycloalkyl, heteroaryl, alkenyl, alkynyl, amido, alkoxy, alkylamino, alkylhydroxy, halo, hydroxyl, carboxylate, alkylcarboxylate, acylazido, sulfonamide or alkyl halo),
or a pharmaceutically acceptable salt thereof,
for use in treating acute neural injury, vascular dementia, or central nervous system (CNS) lupus in a subject in need thereof.

7. The compound for use of claim 6, wherein said compound is:

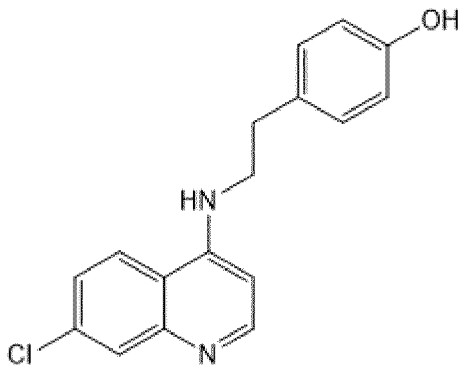

4-{2-[(7-chloroquinolin-4-yl)amino]ethyl}phenol, or

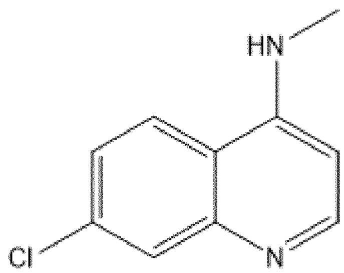

7-chloro-N-methylquinolin-4-amine ,
or a pharmaceutically acceptable salt thereof.

**8.** The compound for use of claim 1, wherein said compound is:

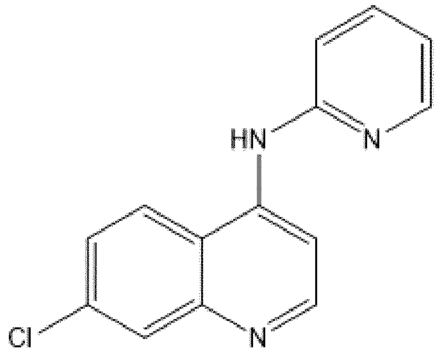

7-chloro-N-(pyridin-2-yl)quinolin-4-amine ,

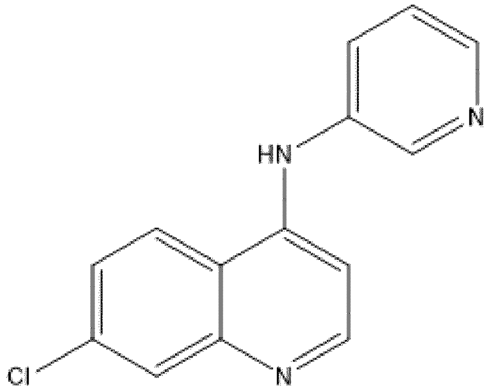

7-chloro-N-(pyridin-3-yl)quinolin-4-amine , or

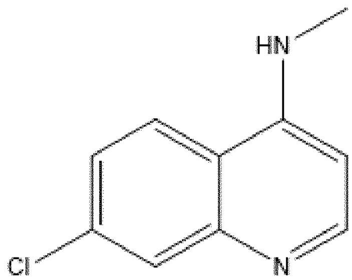

7-chloro-N-methylqiunolin-4-amine ,
or a pharmaceutically acceptable salt thereof.

**9.** A compound of Formula II:

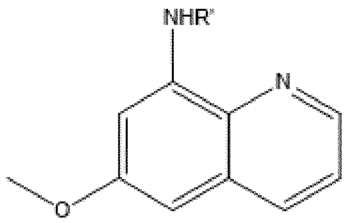

II

wherein R' is selected from the group consisting of pyridin-2-ylmethyl, pyridin-3-ylmethyl, 1-benzylpiperidin-4-yl, 4-cyano-2,2-diethylbutyl, 2-chlorocyclopentyl, 4-(diethylamino)butan-2-yl, 1-(furan-2-yl)ethyl, 1-cyclopropylethyl, 1-ethylpiperidin-4-yl, 5-amino-2,2-diethylpentyl, and 2-(diethylphosphoryl)-1-methylethyl,

or a pharmaceutically acceptable salt thereof,
for use in treating acute neural injury, vascular dementia, or central nervous system (CNS) lupus in a subject in need thereof.

**10.** The compound for use of claim 9, wherein said compound is:

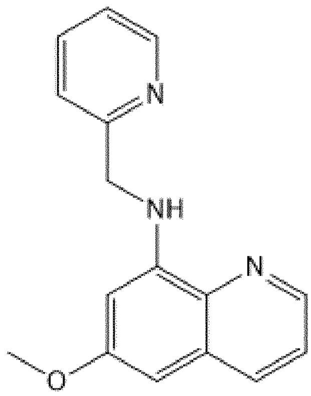

6-methoxy-N-(pyridin-2-ylmethyl)quinolin-8-amine , or

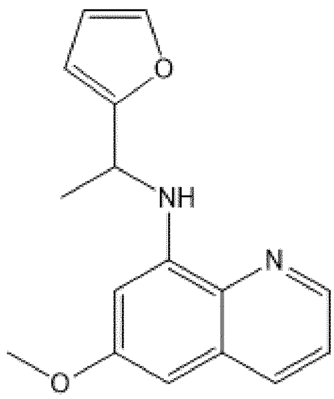

N-[1-(furan-2-yl)ethyl]-6-methoxyquinolin-8-amine ,
or a pharmaceutically acceptable salt thereof.

**11.** The compound for use of any preceding claim, wherein the use is in treating acute neural injury in a subject in need thereof.

**12.** The compound for use of claim 11, wherein said acute neural injury comprises traumatic brain injury or subarachnoid

hemorrhage.

**13.** The compound for use of claim 11, wherein said acute neural injury comprises post-operative cognitive deficit.

**14.** The compound for use of claim 11, wherein said acute neural injury comprises hypoxic brain injury or ischemic brain injury.

**15.** The compound for use of any one of claims 1-10, wherein the method treats vascular dementia in a subject in need thereof.

**16.** The compound for use of any one of claims 1-10, wherein the method treats central nervous system (CNS) lupus in a subject in need thereof.

## Patentansprüche

**1.** Verbindung nach Formel I:

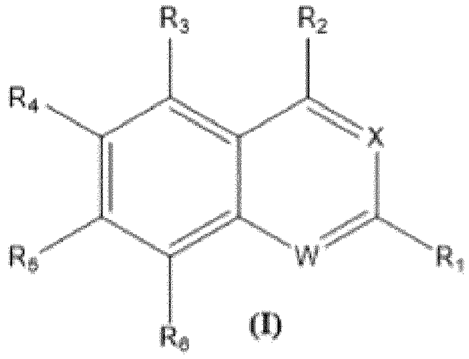

(I)

wobei:

WNist
X $CR_{14}$ ist;
$R_1$ H oder Trifluormethyl ist;
$R_2$ $NR_7R_8$, $OR_{11}$, $SR_{12}$ oder Alkyl ist;
$R_3$ H oder $OR_{13}$ ist;
$R_4$ H oder Methoxy ist;
$R_5$ H, Cl oder Trifluormethyl ist;
$R_6$ H oder Trifluormethyl ist;
$R_7$ H, $C_{1-5}$-Alkyl, Heteroarylalkyl, Cycloalkyl, Heterocycloalkyl, Heterocyclo, Aryl, Heteroaryl, Ureido, Thioureido, Alkenyl, Alkinyl, Amido, Amino, Alkoxy, Alkylamino, Alkylphosphonat, Alkylnitril oder Alkylhalogen, wahlweise substituiert mit $C_{1-5}$-Alkyl, Cycloalkyl, Cycloalkenyl, Heterocycloalkyl, Heteroaryl, Alkenyl, Alkinyl, Amido, Alkoxy, Alkylamino, Alkylhydroxy, Halogen, Hydroxyl, Carboxylat, Alkylcarboxylat, Acylazido, Sulfonamid oder Alkylhalogen ist;
$R_8$ H, $C_{1-5}$-Alkyl, Cycloalkyl, Heterocycloalkyl, Aryl, Heteroaryl, Ureido, Thioureido, Alkenyl, Alkinyl, Amido, Amino, Alkoxy, Alkylamino, Alkylphosphonat, Alkylnitril oder Alkylhalogen, wahlweise substituiert mit $C_{1-5}$-Alkyl, Cycloalkyl, Cycloalkenyl, Heterocycloalkyl, Heteroaryl, Alkenyl, Alkinyl, Amido, Alkoxy, Alkylhydroxy, Halogen, Hydroxyl, Carboxylat, Alkylcarboxylat, Acylazido, Sulfonamid oder Alkylhalogen ist;
$R_9$ H, O, $C_{1-5}$-Alkyl, Cycloalkyl, Heterocycloalkyl, Aryl, Heteroaryl, Alkylamino, Alkylnitril oder Alkylphosphonat ist, wahlweise substituiert mit $C_{1-5}$-Alkyl, Cycloalkyl, Heterocycloalkyl, Aryl, Heteroaryl oder Alkylamino;
$R_{10}$ H, O, $C_{1-5}$-Alkyl, Cycloalkyl, Heterocycloalkyl, Aryl, Heteroaryl, Alkylamino, Alkylnitril oder Alkylphosphonat ist, wahlweise substituiert mit $C_{1-5}$-Alkyl, Cycloalkyl, Heterocycloalkyl, Heteroaryl oder Alkylamino;
$R_{11}$ Alkyl, Aryl oder Heteroaryl ist, wahlweise substituiert mit Alkyl, Halogenalkyl, Aryl oder Heteroaryl;
$R_{12}$ Alkyl, Aryl oder Heteroaryl ist, wahlweise substituiert mit Alkyl, Halogenalkyl, Aryl oder Heteroaryl;
$R_{13}$ Alkyl oder Aryl ist, wahlweise substituiert mit Alkyl oder Haloalkyl; und
$R_{14}$ H oder Aryl ist;

oder ein pharmazeutisch akzeptables Salz davon, zur Verwendung bei der Behandlung von akuter Nervenverletzung, vaskulärer Demenz oder Lupus des zentralen Nervensystems (ZNS) bei einem Patienten, der dies benötigt.

**2.** Verbindung nach Anspruch 1, wobei $R_{14}$ H ist.

**3.** Verbindung zur Verwendung nach einem der vorhergehenden Ansprüche, wobei $R_2$ $NR_7R_8$ ist, $R_7$ H ist und $R_8$ $C_{1-5}$-Alkyl ist, das mit Heteroaryl substituiert ist.

**4.** Verbindung zur Verwendung nach einem der vorhergehenden Ansprüche, wobei $R_1$, $R_3$, $R_4$, und/oder $R_6$ H ist/sind.

**5.** Verbindung zur Verwendung nach einem der vorhergehenden Ansprüche, wobei $R_5$ Cl ist.

**6.** Verbindung nach Formel I(a):

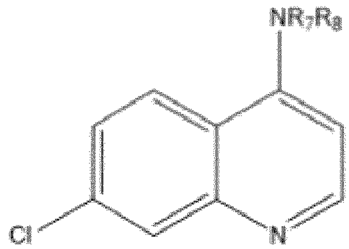

I(a)

wobei $R_7$ und $R_8$ jeweils unabhängig voneinander H oder $C_{1-5}$-Alkyl sind, wobei das $C_{1-5}$-Alkyl wahlweise mit Cycloalkyl, Heterocycloalkyl, Heterocyclo, Aryl oder Heteroaryl substituiert ist (das ferner mit $C_{1-5}$-Alkyl, Cycloalkyl, Cycloalkenyl, Heterocycloalkyl, Heteroaryl, Alkenyl, Alkinyl, Amido, Alkoxy, Alkylamino, Alkylhydroxy, Halogen, Hydroxyl, Carboxylat, Alkylcarboxylat, Acylazido, Sulfonamid oder Alkylhalogen substituiert sein kann), oder ein pharmazeutisch akzeptables Salz davon, zur Verwendung bei der Behandlung von akuter Nervenverletzung, vaskulärer Demenz oder Lupus des zentralen Nervensystems (ZNS) bei einem Patienten, der dies benötigt.

**7.** Verbindung zur Verwendung nach Anspruch 6, wobei die Verbindung Folgendes ist:

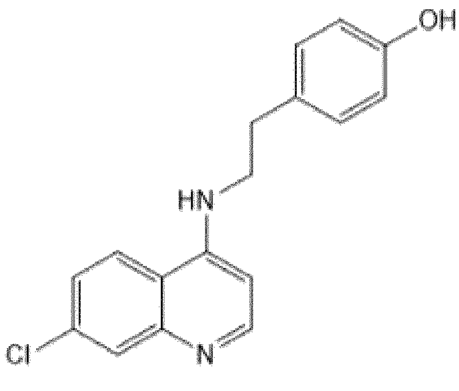

4-{2-[(7-Chlorchinolin-4-yl)amino]ethyl}phenol, oder

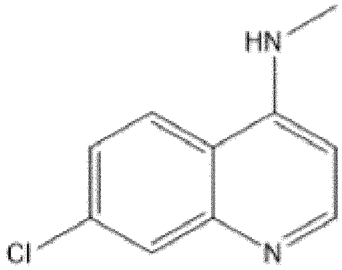

7-Chlor-N-methylchinolin-4-amin,
oder ein pharmazeutisch akzeptables Salz davon.

**8.** Verbindung zur Verwendung nach Anspruch 1, wobei die Verbindung Folgendes ist:

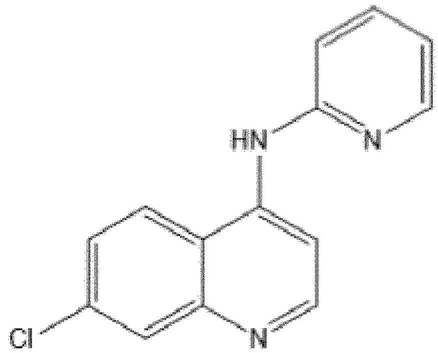

7-Chlor-N-(pyridin-2-yl)chinolin-4-amin,

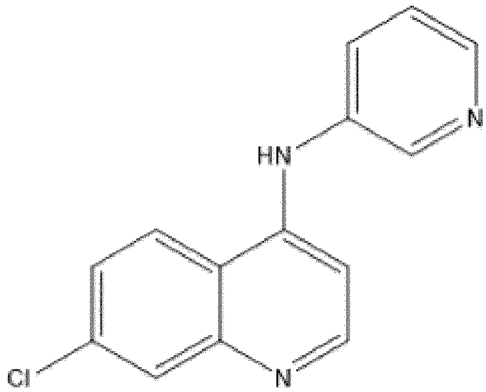

7-Chlor-N-(pyridin-3-yl)chinolin-4-amin, oder

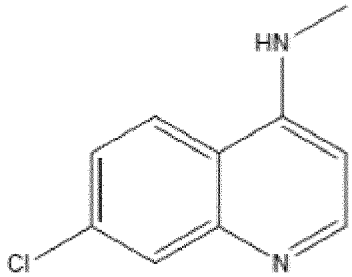

7-Chlor-N-methylchinolin-4-amin,
oder ein pharmazeutisch akzeptables Salz davon.

**9.** Verbindung nach Formel II:

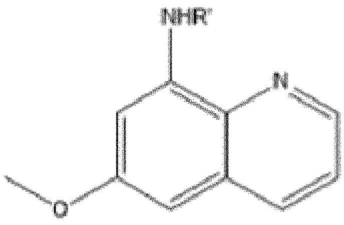

II

wobei R' ausgewählt ist aus der Gruppe, bestehend aus Pyridin-2-ylmethyl, Pyridin-3-ylmethyl, 1-Benzylpiperidin-4-yl, 4-Cyano-2,2-diethylbutyl, 2-Chlorocyclopentyl, 4-(Diethylamino)butan-2-yl, 1-(Furan-2-yl)ethyl, 1-Cyclopropylethyl, 1-Ethylpiperidin-4-yl, 5-Amino-2,2-diethylpentyl und 2-(Diethylphosphoryl)-1-Methylethyl, oder ein pharmazeutisch akzeptables Salz davon,
zur Verwendung bei der Behandlung von akuter Nervenverletzung, vaskulärer Demenz oder Lupus des zentralen Nervensystems (ZNS) bei einem Patienten, der dies benötigt.

**10.** Verbindung zur Verwendung nach Anspruch 9, wobei die Verbindung Folgendes ist:

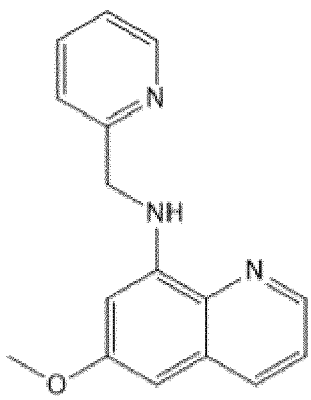

6-Methoxy-N-(pyridin-2-ylmethyl)chinolin-8-amin, oder

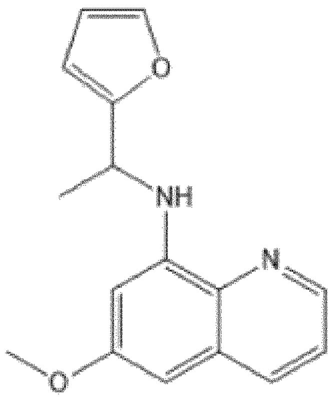

N-[1-(Furan-2-yl)ethyl]-6-methoxychinolin-8-amin,
oder ein pharmazeutisch akzeptables Salz davon.

**11.** Verbindung zur Verwendung nach einem der vorhergehenden Ansprüche, wobei die Verwendung in der Behandlung einer akuten Nervenverletzung bei einer Person, die diese benötigt, besteht.

**12.** Verbindung zur Verwendung nach Anspruch 11, wobei die akute Nervenverletzung eine traumatische Hirnverletzung oder eine Subarachnoidalblutung umfasst.

**13.** Verbindung zur Verwendung nach Anspruch 11, wobei die akute Nervenverletzung ein postoperatives kognitives Defizit umfasst.

**14.** Verbindung zur Verwendung nach Anspruch 11, wobei die akute Nervenverletzung eine hypoxische Hirnverletzung oder eine ischämische Hirnverletzung umfasst.

**15.** Verbindung zur Verwendung nach einem der Ansprüche 1 bis 10, wobei das Verfahren vaskuläre Demenz bei einem Patienten behandelt, der dies benötigt

**16.** Verbindung zur Verwendung nach einem der Ansprüche 1 bis 10, wobei das Verfahren Lupus des zentralen Nervensystems (ZNS) bei einem Patienten behandelt, der dies benötigt.

## Revendications

**1.** Composé de Formule I :

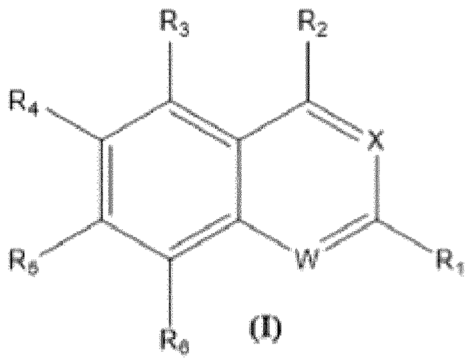

(I)

dans lequel :

W est un N ;
X est un $CR_{14}$ ;
$R_1$ est un H ou un trifluorométhyle ;
$R_2$ est un $NR_7R_8$, $OR_{11}$, $SR_{12}$ ou un alkyle ;
$R_3$ est un H ou $OR_{13}$ ;
$R_4$ est un H ou un méthoxy ;
$R_5$ est un H, Cl ou un trifluorométhyle ;
$R_6$ est un H ou un trifluorométhyle ;
$R_7$ est un H, un alkyle en $C_{1-5}$, un hétéroarylalkyle, un cycloalkyle, un hétérocycloalkyle, un hétérocyclo, un aryle, un hétéroaryle, un uréido, un thiouréido, un alcényle, un alcynyle, un amido, un amino, un alcoxy, un alkylamino, un alkylphosphonate, un alkylnitrile, ou un alkylhalo facultativement substitué par un alkyle en $C_{1-5}$, un cycloalkyle, un cycloalcényle, un hétérocycloalkyle, un hétéroaryle, un alcényle, un alcynyle, un amido, un alcoxy, un alkylamino, un alkhylhydroxy, un halo, un hydroxyle, un carboxylate, un alkylcarbonxylate, un acylazido, un sulfonamide ou un alkylhalo ;
$R_8$ est un H, un alkyle en $C_{1-5}$, un cycloalkyle, un hétérocycloalkyle, un aryle, un hétéroaryle, un uréido, un thiouréido, un alcényle, un alcynyle, un amido, un amino, un alcoxy, un alkylamino, un alkylphosphonate, un alkylnitrile, ou un alkylhalo facultativement substitué par un alkyle en $C_{1-5}$, un cycloalkyle, un cycloalcényle, un hétérocycloalkyle, un hétéroaryle, un alcényle, un alcynyle, un amido, un alcoxy, un alkhylhydroxy, un halo, un hydroxyle, un carboxylate, un alkylcarboxylate, un acylazido, un sulfonamide ou un alkylhalo ;
$R_9$ est un H, un O, un alkyle en $C_{1-5}$, un cycloalkyle, un hétérocycloalkyle, un aryle, un hétéroaryle, un alkylamino, un alkylnitrile, ou un alkylphosphonate facultativement substitué par un alkyle en $C_{1-5}$, un cycloalkyle, un hétérocycloalkyle, un aryle, un hétéroaryle ou un alkylamino ;
$R_{10}$ est un H, un O, un alkyle en $C_{1-5}$, un cycloalkyle, un hétérocycloalkyle, un aryle, un hétéroaryle, un alkylamino,

un alkylnitrile, ou un alkylphosphonate facultativement substitué par un alkyle en $C_{1-5}$, un cycloalkyle, un hétérocycloalkyle, un hétéroaryle ou un alkylamino ;
$R_{11}$ est un alkyle, un aryle ou un hétéroaryle facultativement substitué par un alkyle, un haloalkyle, un aryle ou un hétéroaryle ;
$R_{12}$ est un alkyle, un aryle ou un hétéroaryle facultativement substitué par un alkyle, un haloalkyle, un aryle ou un hétéroaryle ;
$R_{13}$ est un alkyle ou un aryle facultativement substitué par un alkyle ou un haloalkyle ; et
$R_{14}$ est un H ou un aryle ;
ou un sel pharmaceutiquement acceptable de ceux-ci,
pour utilisation dans le traitement de lésions neuronales aiguës, de démence vasculaire, ou d'un lupus du système nerveux central (SNC) chez un sujet en ayant besoin.

**2.** Composé pour utilisation selon la revendication 1, dans lequel $R_{14}$ est un H.

**3.** Composé pour utilisation selon l'une quelconque des revendications précédentes, dans lequel $R_2$ est un $NR_7R_8$, $R_7$ est un H et $R_8$ est un alkyle en $C_{1-5}$ substitué par un hétéroaryle.

**4.** Composé pour utilisation selon l'une quelconque des revendications précédentes, dans lequel $R_1$, $R_3$, $R_4$, et/ou $R_6$ est/sont H.

**5.** Composé pour utilisation selon l'une quelconque des revendications précédentes, dans lequel $R_5$ est un Cl.

**6.** Composé de Formule I(a) :

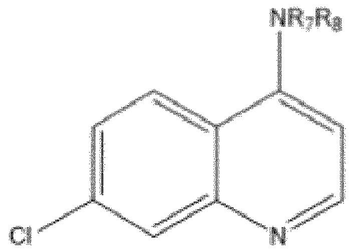

I(a)

dans lequel $R_7$ et $R_8$ sont chacun indépendamment un H ou un alkyle en $C_{1-5}$, dans lequel ledit alkyle en $C_{1-5}$ est facultativement substitué par un cycloalkyle, un hétérocycloalkyle, un hétérocyclo, un aryle ou un hétéroaryle (qui peut être en outre substitué par un alkyle en $C_{1-5}$, un cycloalkyle, un cycloalcényle, un hétérocycloalkyle, un hétéroaryle, un alcényle, un alcynyle, un amido, un alcoxy, un alkylamino, un alkylhydroxy, un halo, un hydroxyle, un carboxylate, un alkylcarboxylate, un acylazido, un sulfonamide ou un alkylhalo),
ou un sel pharmaceutiquement acceptable de ceux-ci,
pour utilisation dans le traitement de lésions neuronales aiguës, de la démence vasculaire, ou d'un lupus du système nerveux central (SNC) chez un sujet en ayant besoin.

**7.** Composé pour utilisation selon la revendication 6, dans lequel ledit composé est :

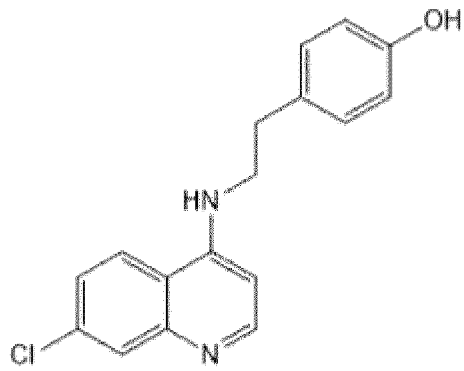

un 4-{2-[(7-chloroquinolin-4-yl)amino]éthyl}phénol, ou

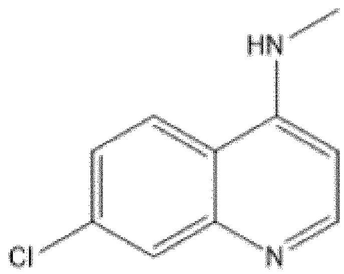

une 7-chloro-N-méthylquinolin-4-amine,
ou un sel pharmaceutiquement acceptable de celui-ci.

**8.** Composé pour utilisation selon la revendication 1, dans lequel ledit composé est :

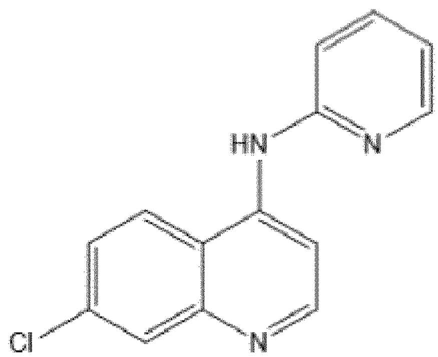

une 7-chloro-N-(pyridin-2-yl)quinolin-4-amine,

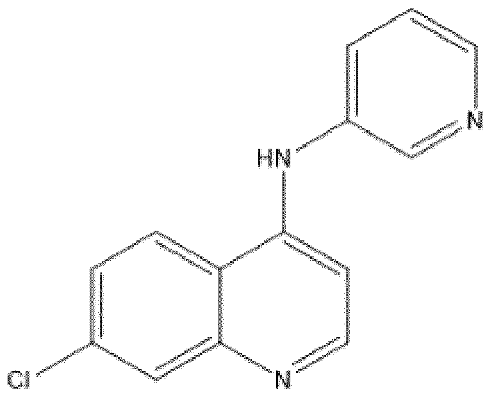

une 7-chloro-N-(pyridin-3-yl)quinolin-4-amine, ou

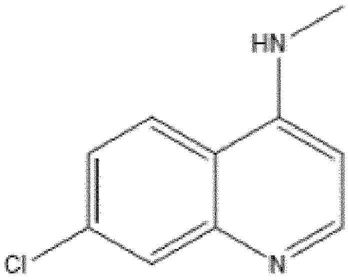

une 7-chloro-N-méthylquinolin-4-amine,
ou un sel pharmaceutiquement acceptable de celui-ci.

**9.** Composé de Formule II :

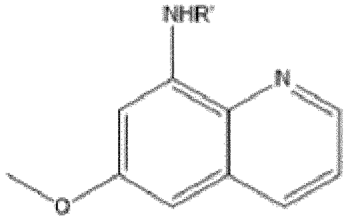

II

dans lequel R' est sélectionné dans le groupe constitué de pyridin-2-ylméthyle, pyridin-3-ylméthyle, 1-benzylpipéridin-4-yle, 4-cyano-2,2-diéthylbutyle, 2-chlorocyclopentyle, 4-diéthylamino)butan-2-yle, 1-(furan-2-yl)éthyle, 1-cyclopropyléthyle, 1-éthylpipéridin-4-yle, 5-amino-2,2-diéthylpentyle, et 2-(diéthylphosphoryl)-1-méthyléthyle,
ou un sel pharmaceutiquement acceptable de celui-ci,
pour utilisation dans le traitement de lésions neuronales aiguës, de la démence vasculaire, ou d'un lupus du système nerveux central (SNC) chez un sujet en ayant besoin.

**10.** Composé pour utilisation selon la revendication 9, dans lequel ledit composé est :

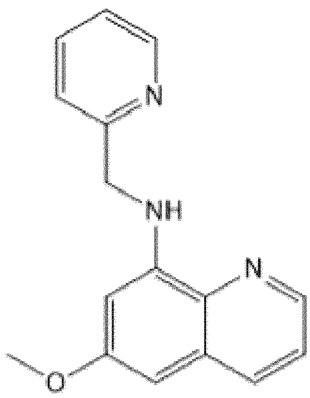

une 6-méthoxy-N-(pyridin-2-ylméthyl)quinolin-8-amine ; ou

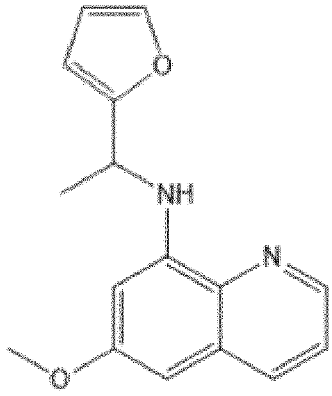

une N-[1-furan-2-yl)éthyl]-6-méthoxyquinolin-8-amine,
ou un sel pharmaceutiquement acceptable de celui-ci.

**11.** Composé pour utilisation selon l'une quelconque des revendications précédentes, dans lequel l'utilisation consiste à traiter une lésion neuronale aiguë chez un sujet en ayant besoin.

**12.** Composé pour utilisation selon la revendication 11, dans lequel ladite lésion neuronale aiguë comprend une lésion cérébrale traumatique ou une hémorragie sous-arachnoïdienne.

**13.** Composé pour utilisation selon la revendication 11, dans lequel ladite lésion neuronale aiguë comprend un déficit cognitif post-opératoire.

**14.** Composé pour utilisation selon la revendication 11, dans lequel ladite lésion neuronale aiguë comprend une lésion cérébrale hypoxique ou une lésion cérébrale ischémique.

**15.** Composé pour utilisation selon l'une quelconque des revendications 1 à 10, dans lequel le procédé traite la démence vasculaire chez un sujet en ayant besoin.

**16.** Composé pour utilisation selon l'une quelconque des revendications 1 à 10, dans lequel le procédé traite un lupus du système nerveux central (SNC) chez un sujet en ayant besoin.

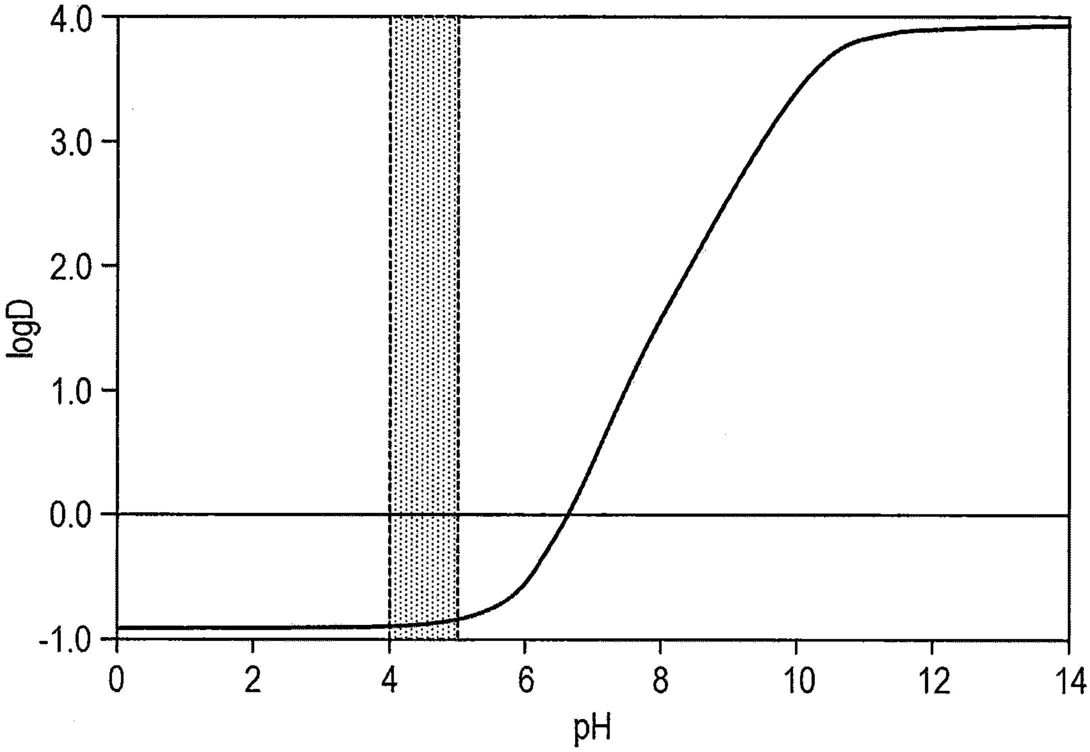
4.0
3.0
2.0
1.0
0.0
-1.0
logD
0
2
4
6
8
10
12
14
pH

FIG. 1A

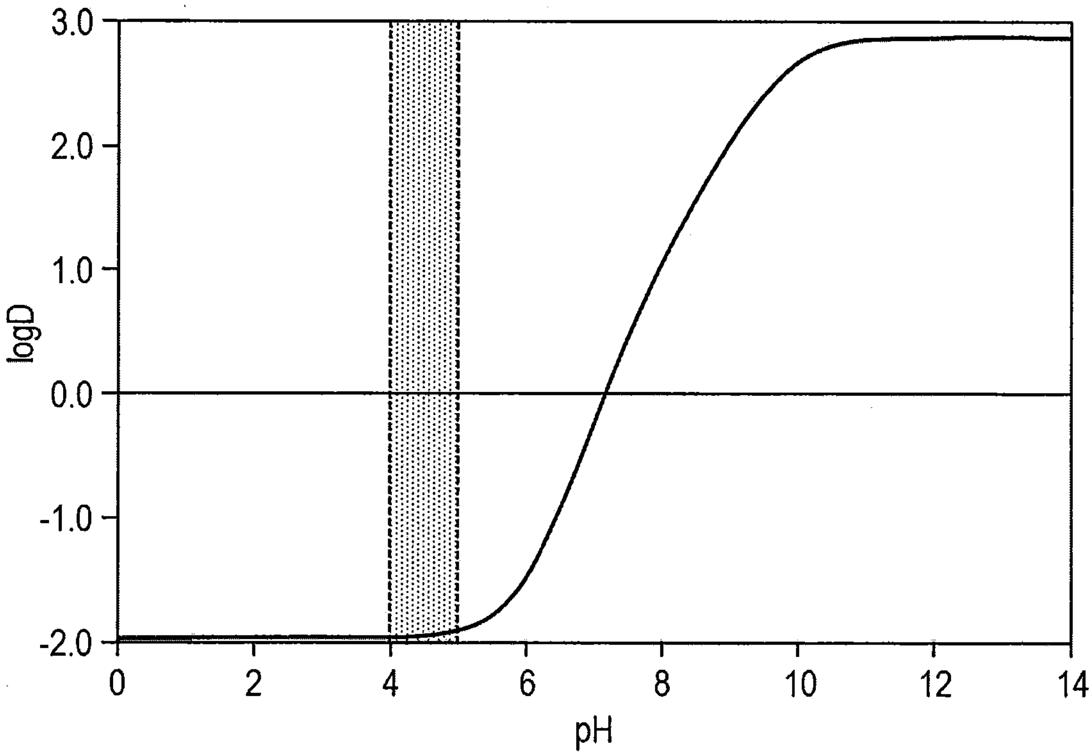
3.0
2.0
1.0
0.0
-1.0
-2.0
logD
0
2
4
6
8
10
12
14
pH

FIG. 1B

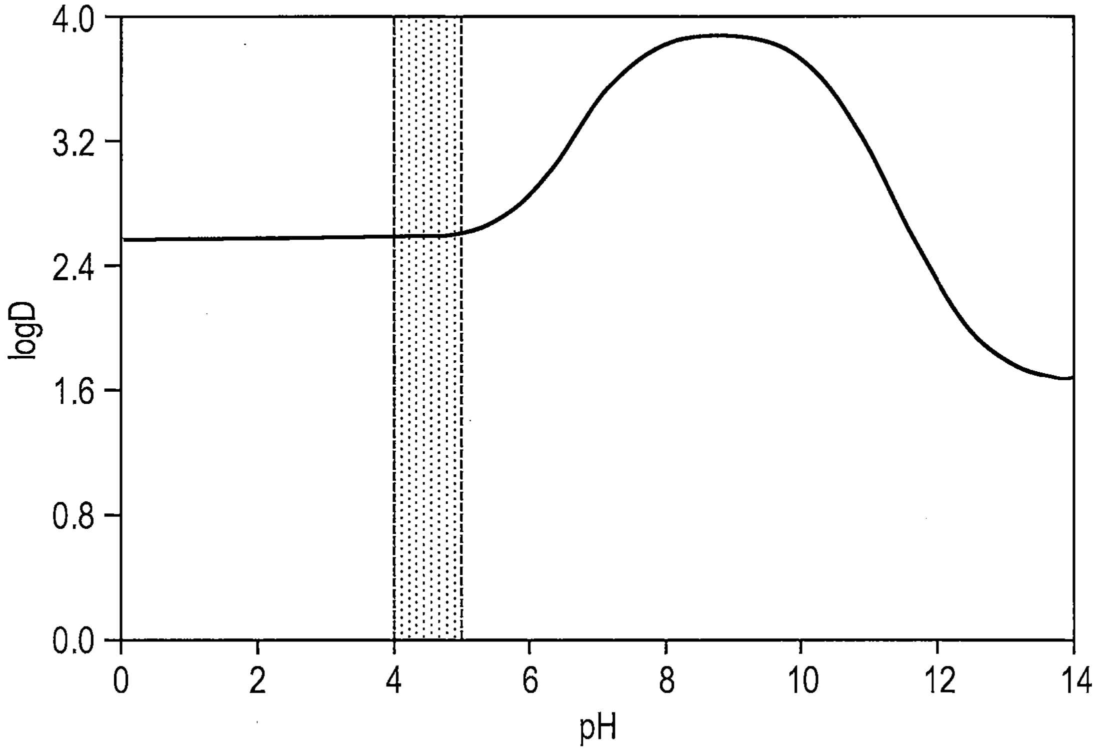
4.0
3.2
2.4
1.6
0.8
0.0
logD
0
2
4
6
8
10
12
14
pH

FIG. 2A

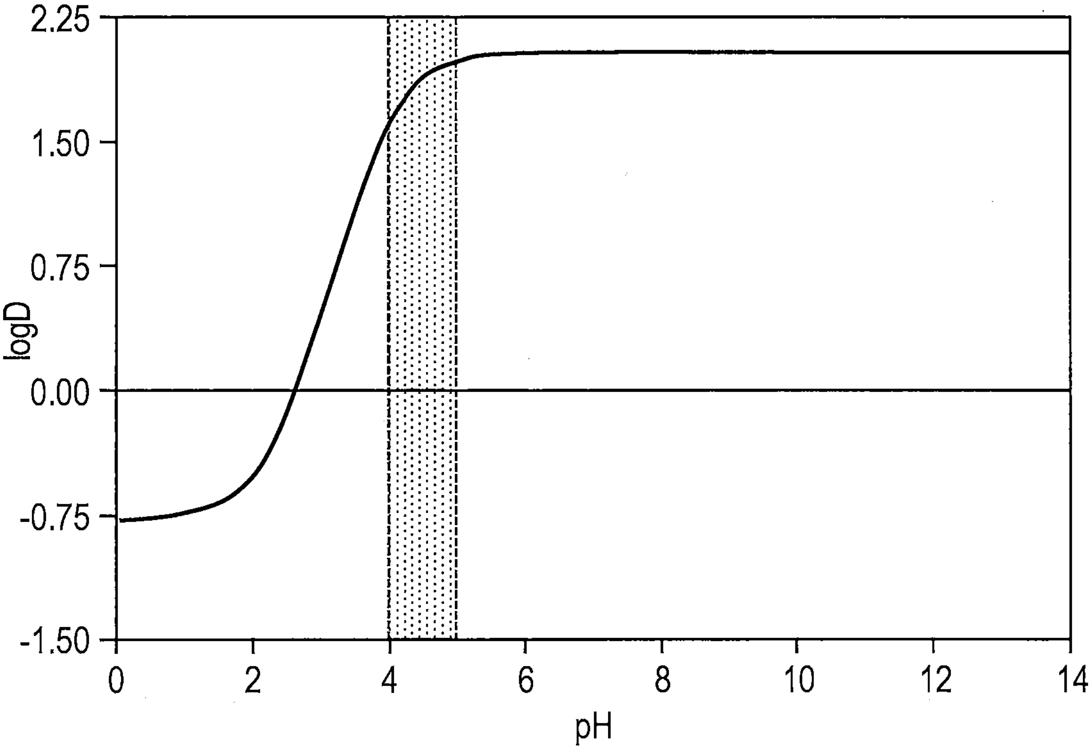
2.25
1.50
0.75
0.00
-0.75
-1.50
logD
0
2
4
6
8
10
12
14
pH

FIG. 2B

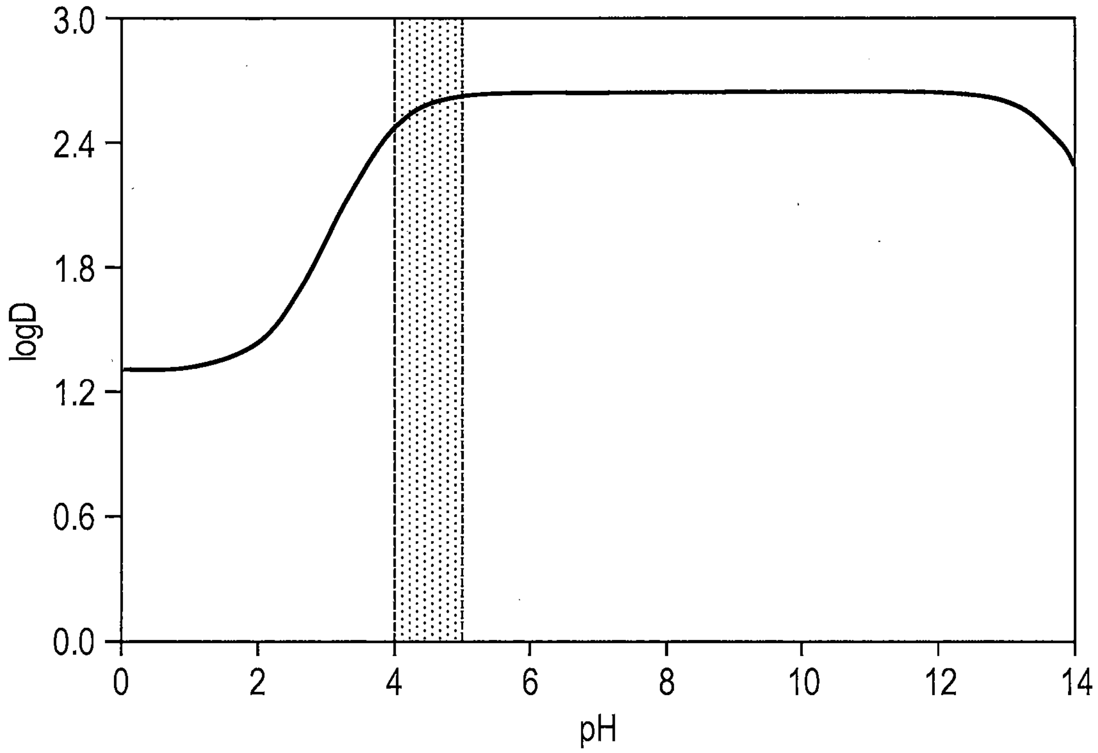
3.0
2.4
1.8
1.2
0.6
0.0
logD
0
2
4
6
8
10
12
14
pH

*FIG. 2C*

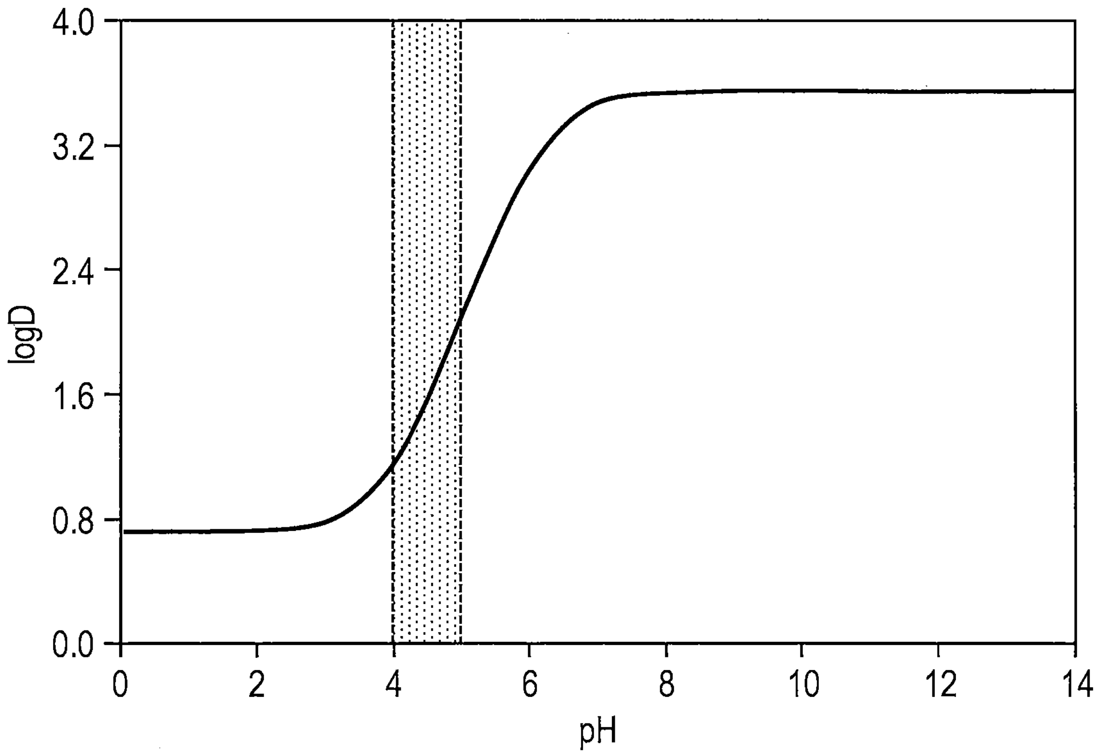
4.0
3.2
2.4
1.6
0.8
0.0
logD
0
2
4
6
8
10
12
14
pH

*FIG. 2D*

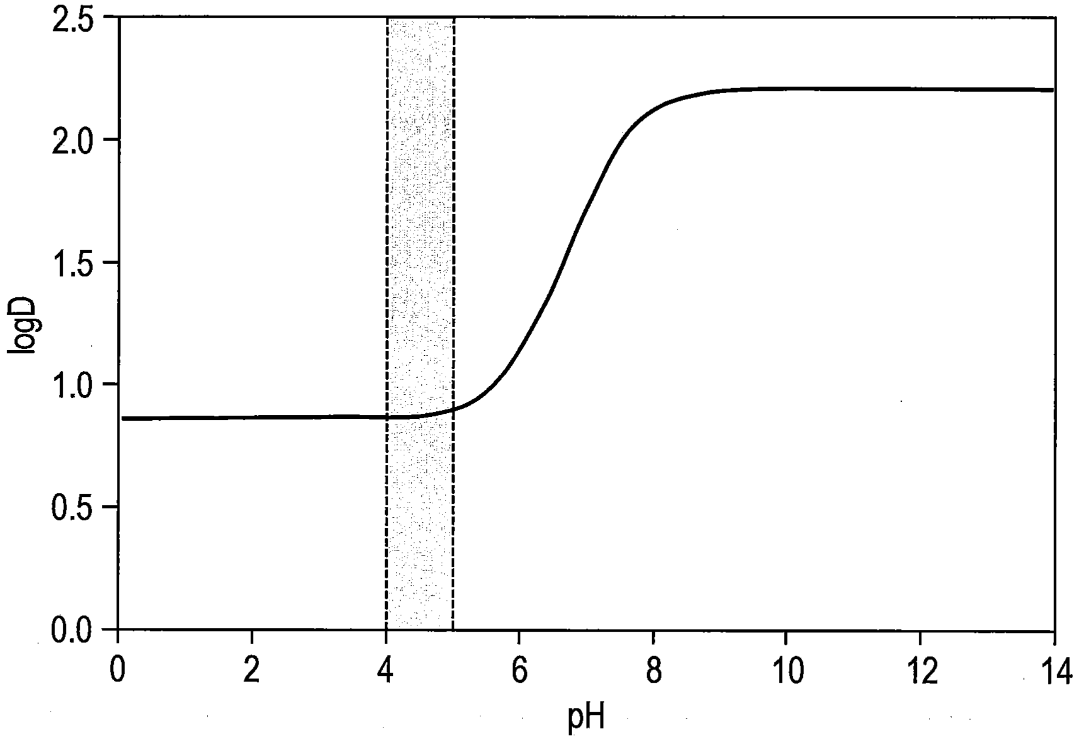


*FIG. 2E*

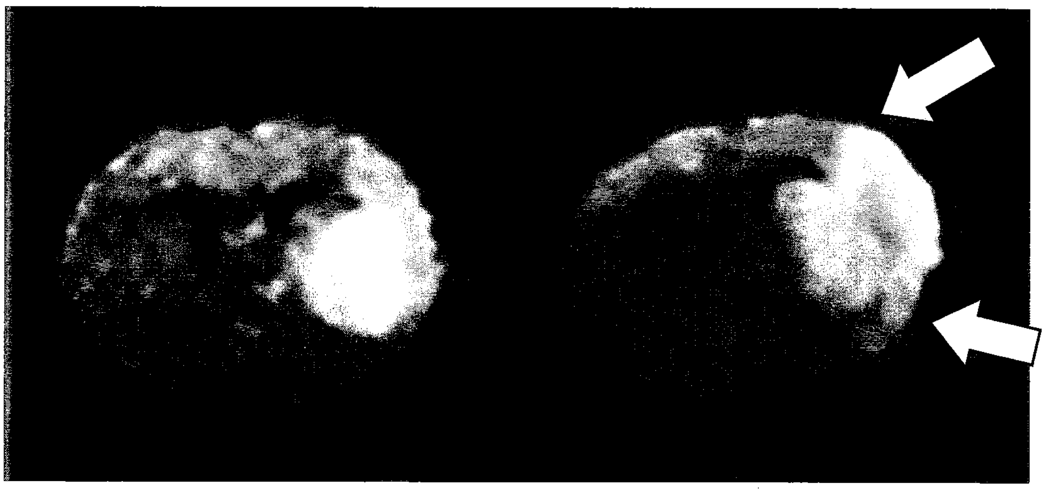

*FIG. 3A*

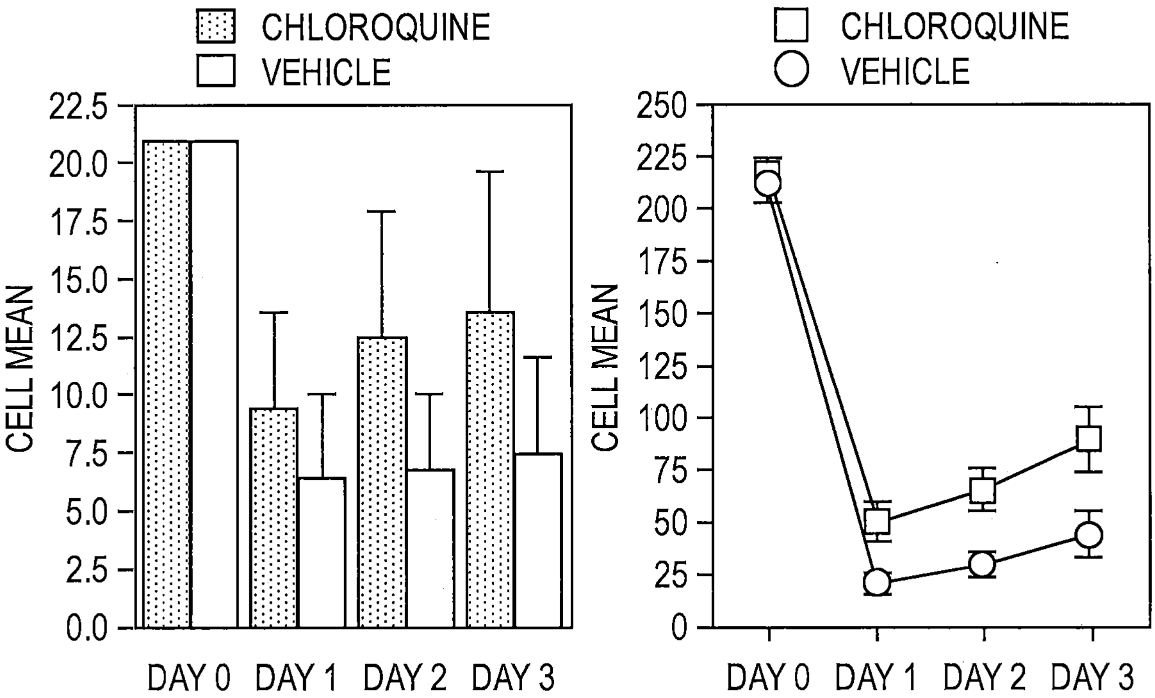
CHLOROQUINE
VEHICLE
CELL MEAN
22.5
20.0
17.5
15.0
12.5
10.0
7.5
5.0
2.5
0.0
DAY 0
DAY 1
DAY 2
DAY 3
CHLOROQUINE
VEHICLE
CELL MEAN
250
225
200
175
150
125
100
75
50
25
0
DAY 0
DAY 1
DAY 2
DAY 3

*FIG. 3B*

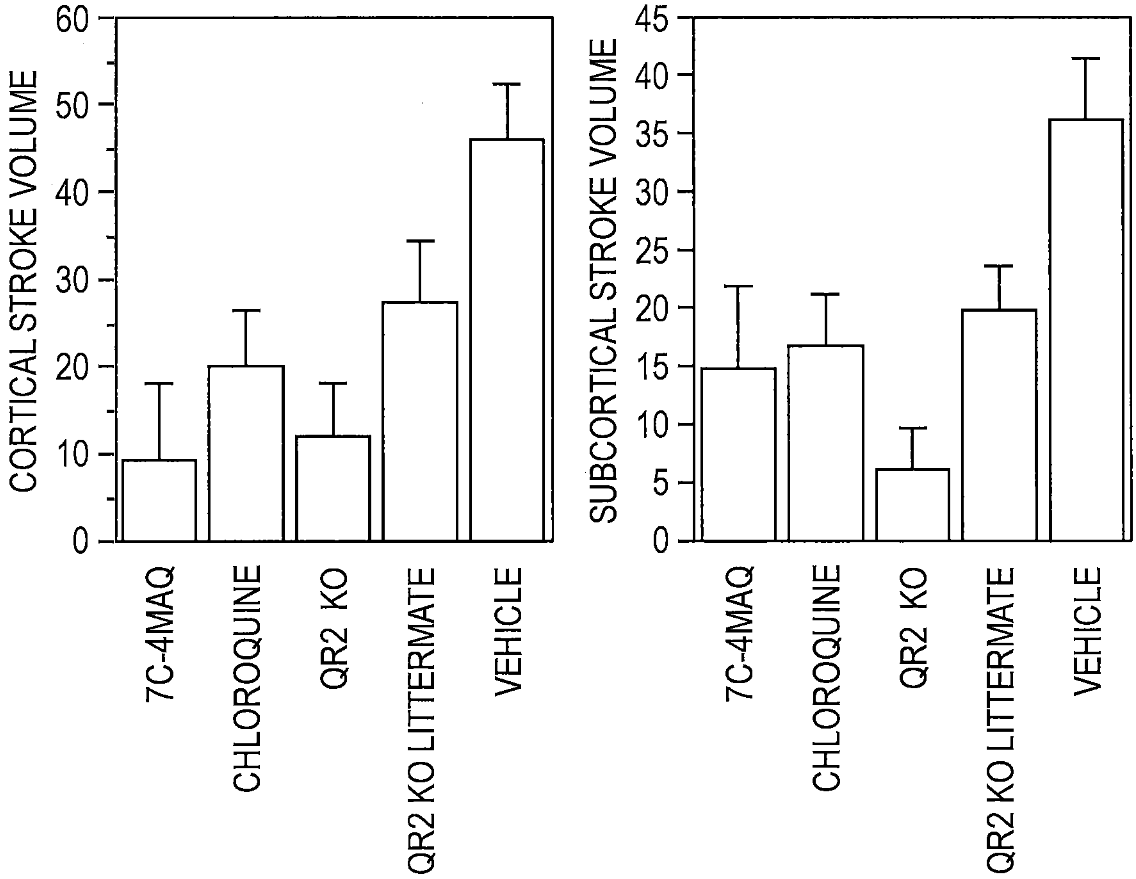
CORTICAL STROKE VOLUME
60
50
40
30
20
10
0
7C-4MAQ
CHLOROQUINE
QR2 KO
QR2 KO LITTERMATE
VEHICLE
SUBCORTICAL STROKE VOLUME
45
40
35
30
25
20
15
10
5
0
7C-4MAQ
CHLOROQUINE
QR2 KO
QR2 KO LITTERMATE
VEHICLE

*FIG. 3C*

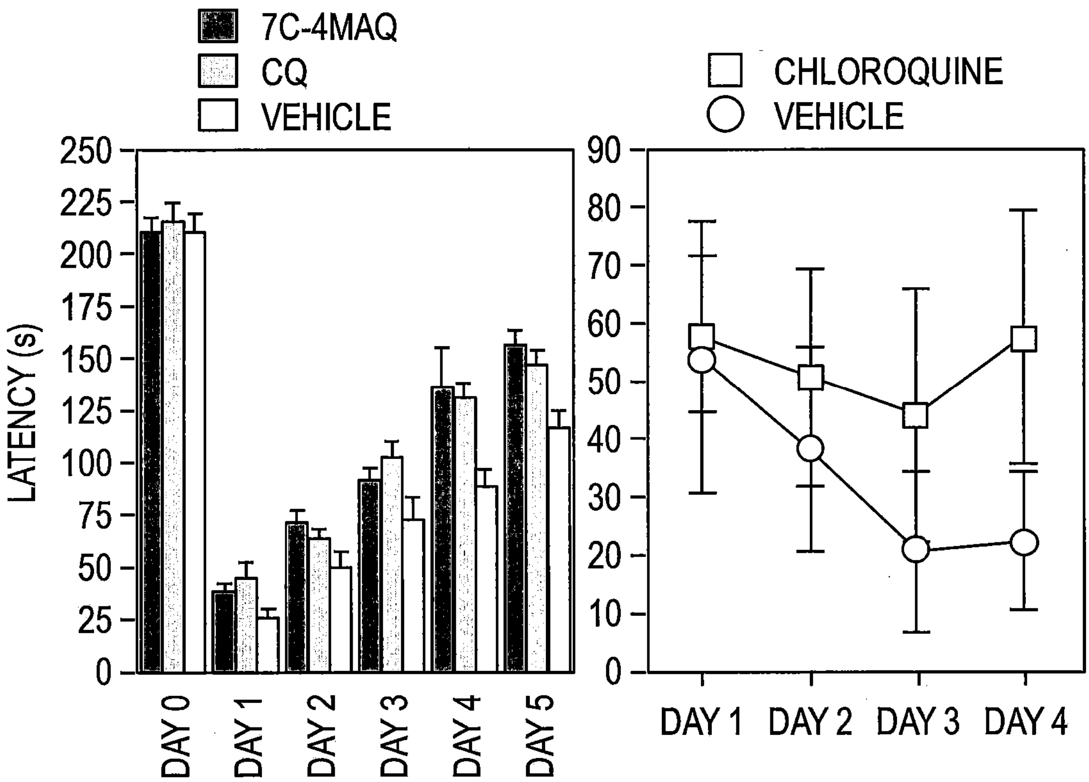
7C-4MAQ
CQ
VEHICLE
LATENCY (s)
250
225
200
175
150
125
100
75
50
25
0
DAY 0
DAY 1
DAY 2
DAY 3
DAY 4
DAY 5
CHLOROQUINE
VEHICLE
90
80
70
60
50
40
30
20
10
0
DAY 1
DAY 2
DAY 3
DAY 4

*FIG. 4*

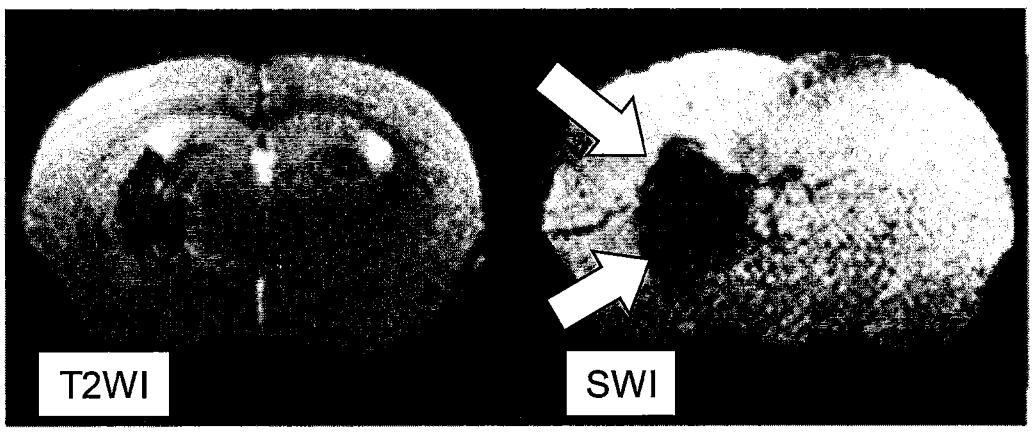
T2WI
SWI

*FIG. 5*

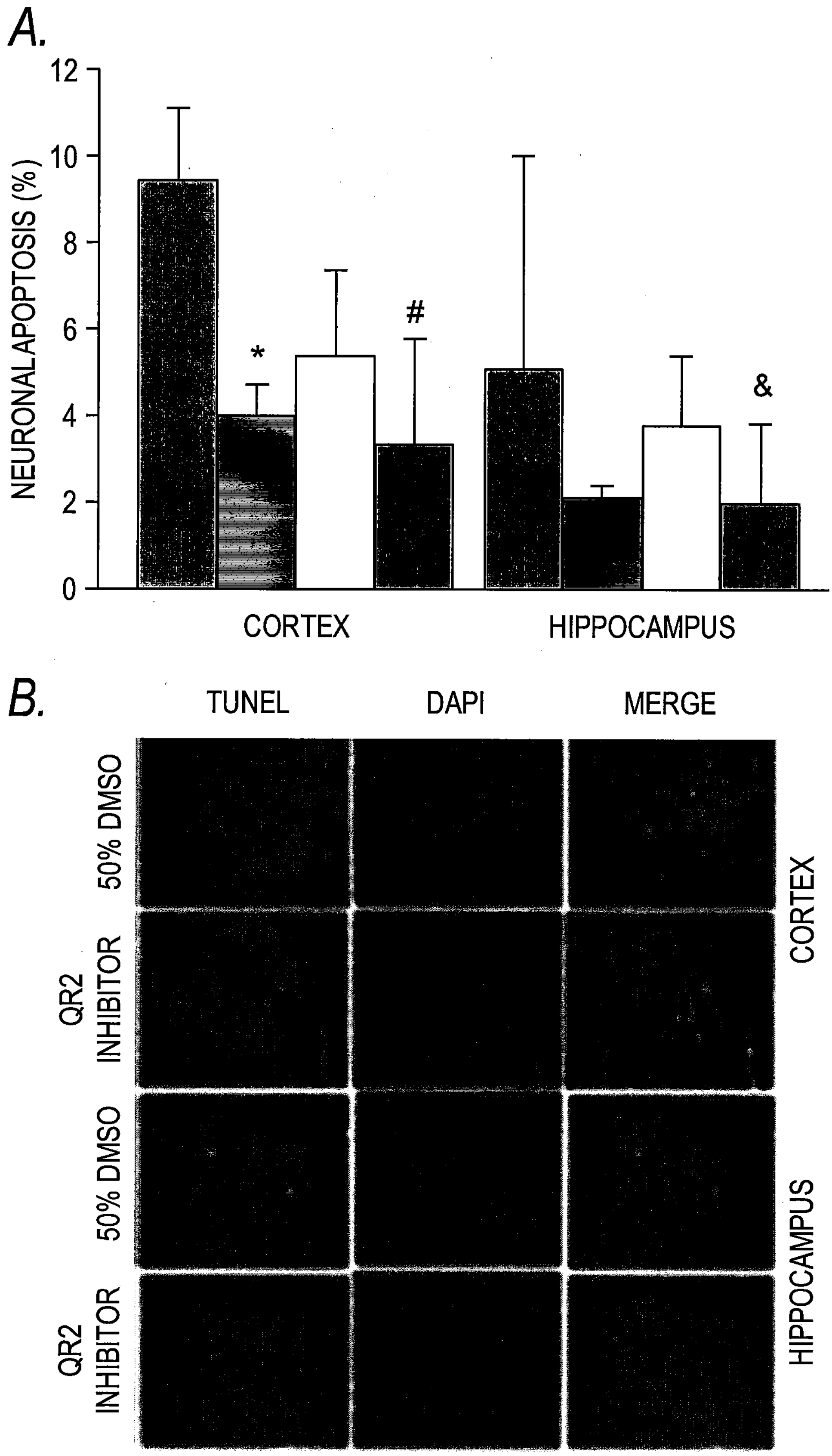
A.
12
10
8
6
4
2
0
NEURONALAPOPTOSIS (%)
*
#
&
CORTEX
HIPPOCAMPUS
B.
TUNEL
DAPI
MERGE
50% DMSO
QR2 INHIBITOR
50% DMSO
QR2 INHIBITOR
CORTEX
HIPPOCAMPUS

FIG. 6

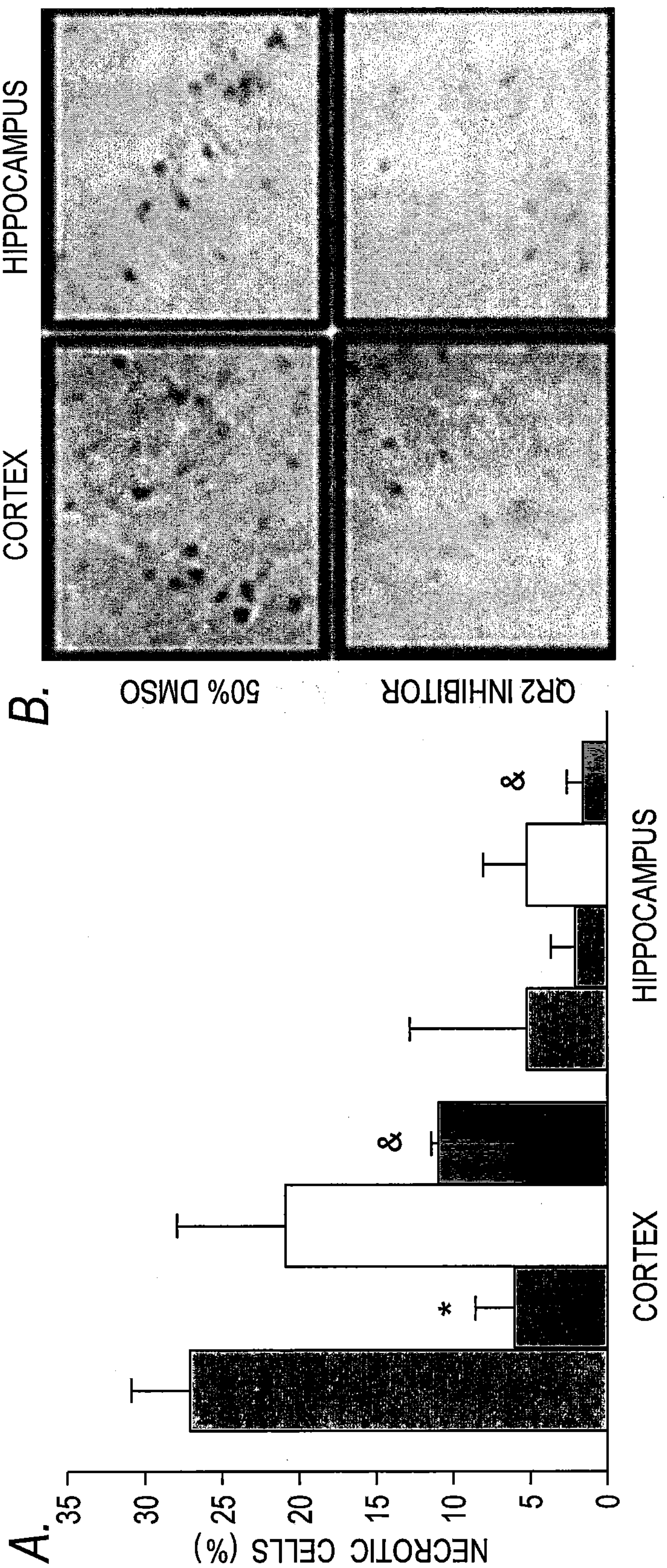
A.
NECROTIC CELLS (%)
35
30
25
20
15
10
5
0
*
&
&
CORTEX
HIPPOCAMPUS
B.
CORTEX
HIPPOCAMPUS
50% DMSO
QR2 INHIBITOR

FIG. 7

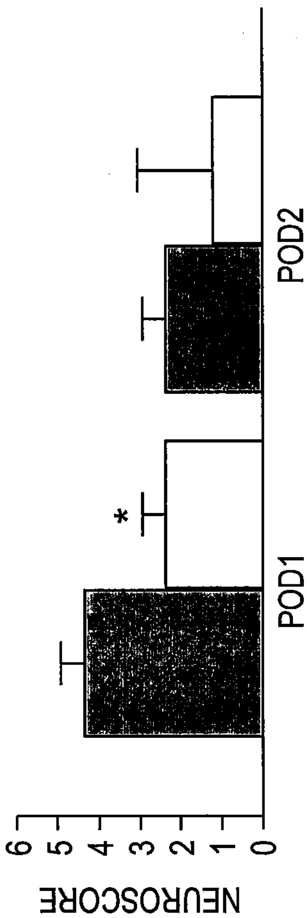
NEUROSCORE
6
5
4
3
2
1
0
*
POD1
POD2

FIG. 8

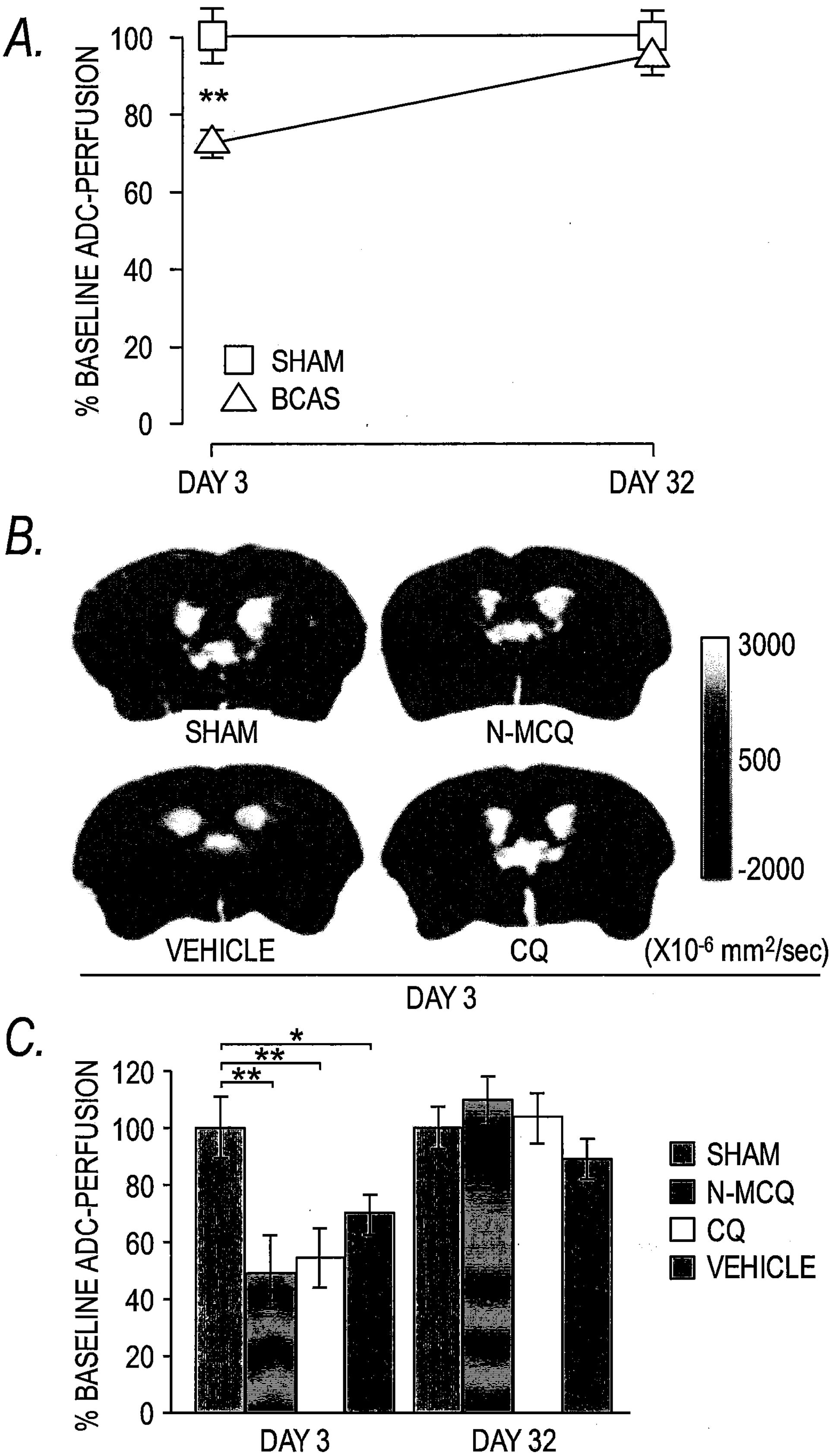
A.
100
80
60
40
20
0
% BASELINE ADC-PERFUSION
**
SHAM
BCAS
DAY 3
DAY 32
B.
SHAM
N-MCQ
VEHICLE
CQ
3000
500
-2000
(X10-6 mm2/sec)
DAY 3
C.
120
100
80
60
40
20
0
% BASELINE ADC-PERFUSION
*
**
**
SHAM
N-MCQ
CQ
VEHICLE
DAY 3
DAY 32

*FIG. 9*

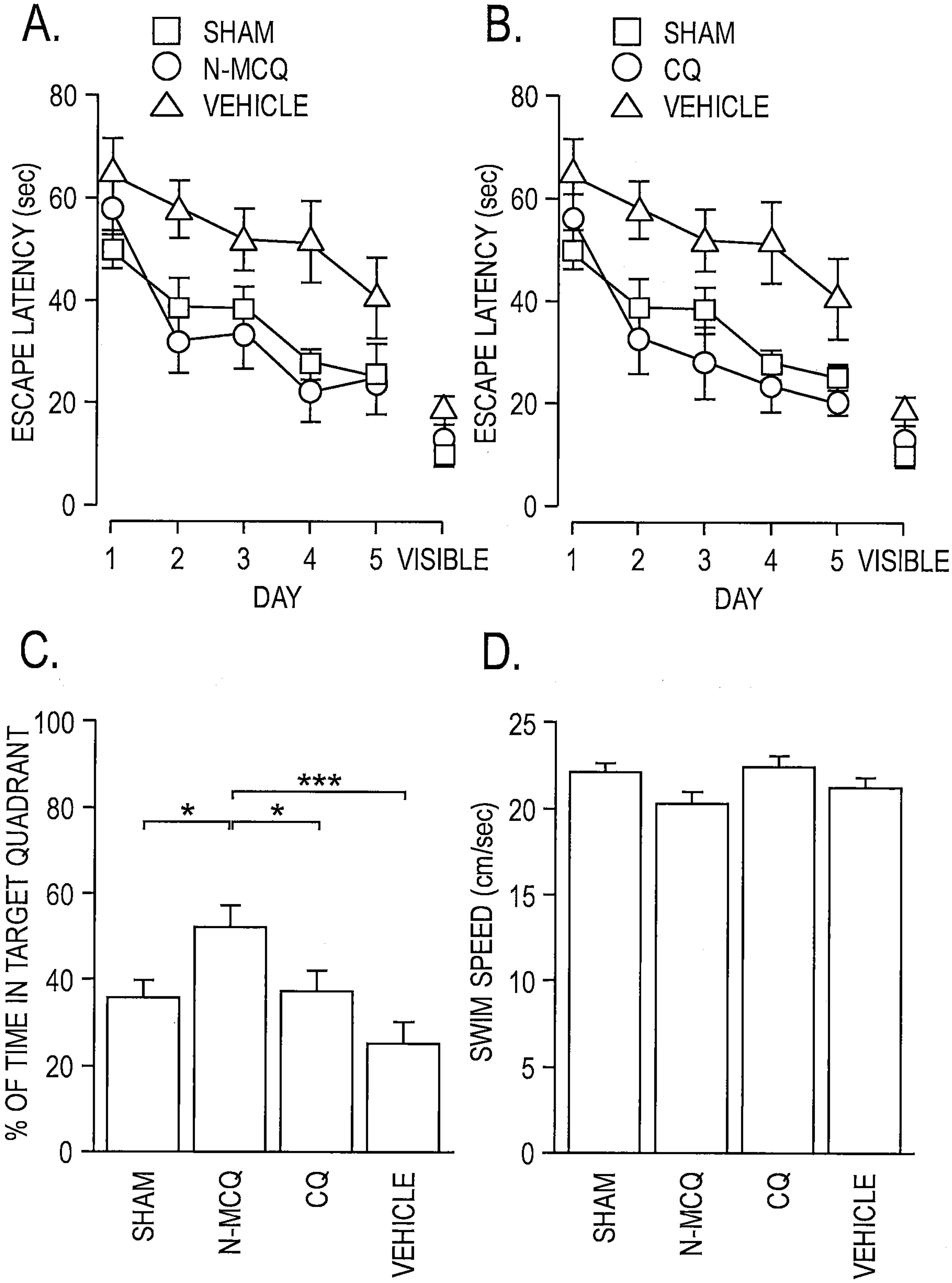
A.
SHAM
N-MCQ
VEHICLE
ESCAPE LATENCY (sec)
80
60
40
20
0
1
2
3
4
5
VISIBLE
DAY
B.
SHAM
CQ
VEHICLE
ESCAPE LATENCY (sec)
DAY
C.
% OF TIME IN TARGET QUADRANT
100
*
***
SHAM
N-MCQ
CQ
VEHICLE
D.
SWIM SPEED (cm/sec)
25
15
10
5
SHAM
N-MCQ
CQ
VEHICLE

*FIG. 10*

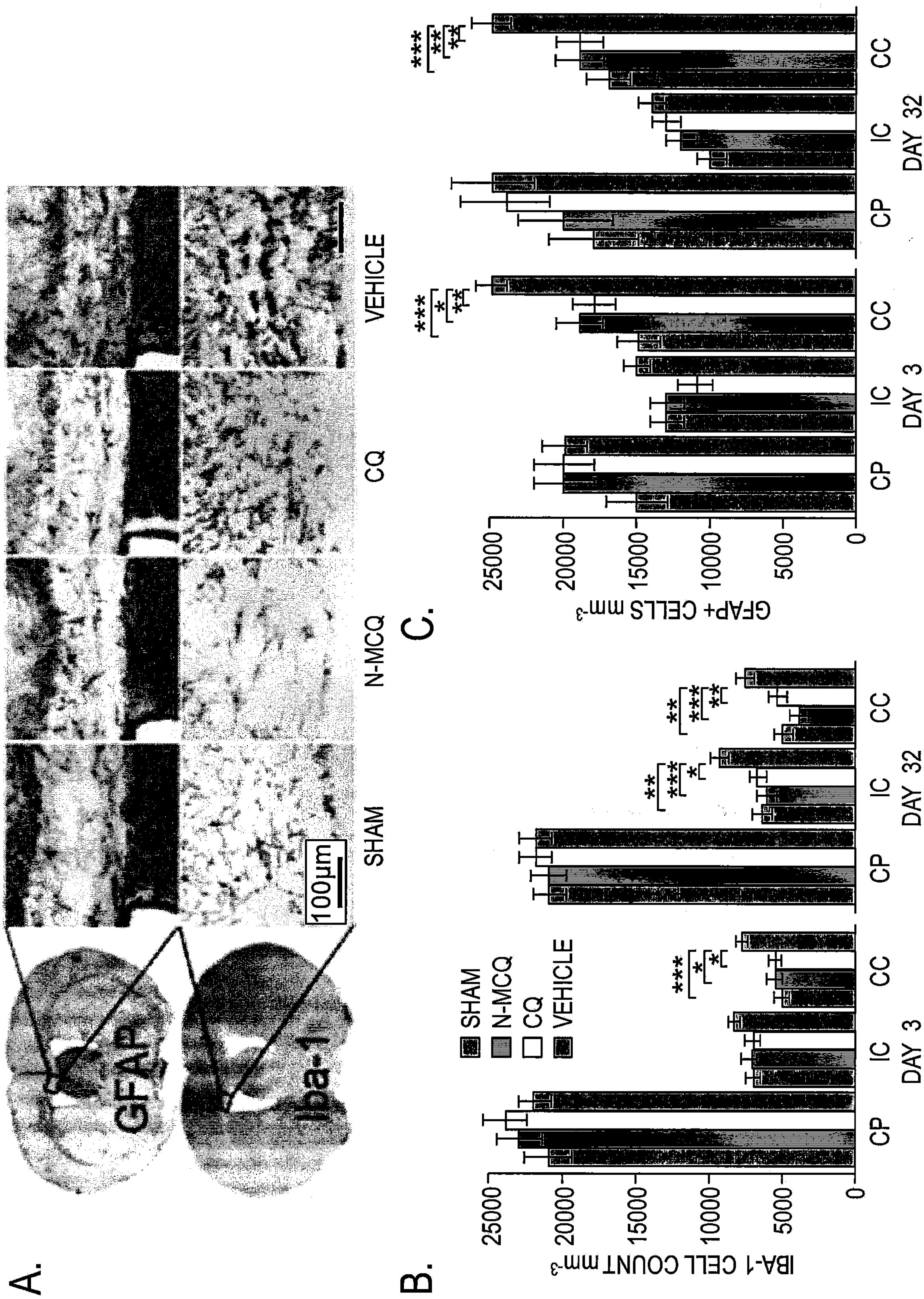
A.
GFAP
Iba-1
100µm
SHAM
N-MCQ
CQ
VEHICLE
B.
IBA-1 CELL COUNT mm-3
25000
20000
15000
10000
5000
0
SHAM
N-MCQ
CQ
VEHICLE
CP
IC
CC
DAY 3
DAY 32
C.
GFAP+ CELLS mm-3
***
**
*

FIG. 11

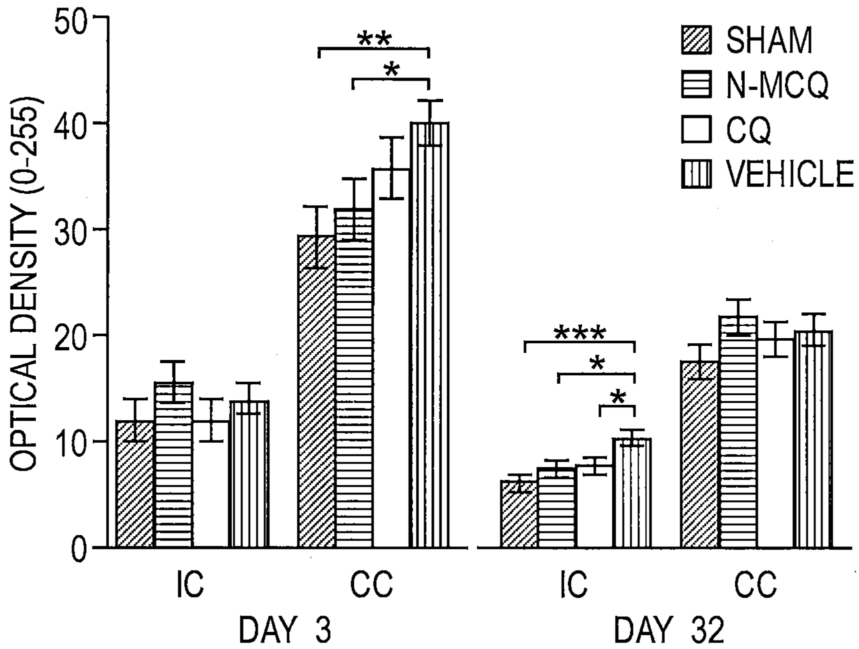
OPTICAL DENSITY (0-255)
50
40
30
20
10
0
SHAM
N-MCQ
CQ
VEHICLE
**
*
***
*
*
IC
CC
DAY 3
IC
CC
DAY 32

*FIG. 12*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060074105 A, Ware
- WO 2008074068 A1
- US 2006074105 A1
- US 2017226095 A1
- US 2012040993 A1
- US 2015335635 A1
- WO 2008014602 A1
- US 2011190342 A1
- US 2012172376 A1
- WO 2014066506 A2
- US 6680299 B
- US 6680324 B
- US 6680322 B
- US 20060074105, Ware, Jr.,
- US 4159331 A, McCall


### Non-patent literature cited in the description


- **SINGER et al.** Update on immunosuppressive therapy. *Curr. Opin. Rheumatol.,* 1998, vol. 10, 169-173
- **WALLACE.** The use of chloroquine and hydroxychloroquine for non-infectious conditions other than rheumatoid arthritis or lupus: a crucial review. *Lupus,* 1996, vol. 1, 59-64
- **SAVARINO et al.** Effects of chloroquine on viral infections: an old drug against today's diseases?. *Lancet Infect. Dis.,* 2003, vol. 3 (11), 722-7
- **SAVARINO et al.** Risks and benefits of chloroquine use in anticancer strategies. *Lancet Oncol.,* 2006, vol. 7 (10), 792-3
- **SOTELO et al.** Adding chloroquine to conventional treatment for glioblastoma multiforme: a randomized, double-blind, placebo-controlled trial. *Ann. Intern. Med.,* 2006, vol. 144 (5), 337-43
- **GIULIAN et al.** The role of mononuclear phagocytes in wound healing after traumatic injury to adult mammalian brain. *J. Neurosci.,* 1989, vol. 9, 4416-4429
- **IVANOVA et al.** Cerebral ischemia enhances polyamine oxidation: identification of enzymatically formed 3-aminopropanal as an endogenous mediator of neuronal and glial cell death. *J. Exp. Med.,* 1998, vol. 188, 327-340
- **GIULIAN.** Microglia and the immune pathology of Alzheimer disease. *Am. J. Hum. Genet.,* 1999, vol. 65, 13-18
- Bioreversible Carriers in Drug Design. **T. HIGUCHI ; V. STELLA.** Prodrugs as Novel delivery Systems. American Pharmaceutical Association and Pergamon Press, 1987, vol. 14
- **EGAN et al.** *J. Med. Chem.,* 2000, vol. 43, 283-291
- **STOCKS et al.** *J. Med. Chem.,* 2002, vol. 45, 4975-4983
- Remington's Pharmaceutical Sciences. Mack Publishing Company, 1990
- **LASKOWITZ et al.** Neuroprotective pentapeptide CN-105 is associated with reduced sterile inflammation and improved functional outcomes in a traumatic brain injury murine model. *Sci. Rep.,* 21 April 2017, vol. 7, 46461
- **LEI et al.** Neuroprotective pentapeptide CN-105 improves functional and histological outcomes in a murine model of intracerebral hemorrhage. *Sci. Rep.,* 07 October 2016, vol. 6, 34834
- **LANGE et al.** A novel survival model of cardioplegic arrest and cardiopulmonary bypass in rats: a methodology paper. *J Cardiothorac Surg.,* 19 August 2008, vol. 3, 51